(19) Europäisches Patentamt
European Patent Office
Office européen des brevets

(11) Publication number: **0 516 861 A1**

# (12) EUROPEAN PATENT APPLICATION published in accordance with Art. 158(3) EPC

(21) Application number: **91920828.0**

(51) Int. Cl.[5]: **C07D 401/04**, C07D 471/04

(22) Date of filing: **29.11.91**

(86) International application number: **PCT/JP91/01640**

(87) International publication number: **WO 92/12146 (23.07.92 92/19)**

(30) Priority: **27.12.90 JP 414635/90**
**05.11.91 JP 317544/91**

(43) Date of publication of application: **09.12.92 Bulletin 92/50**

(84) Designated Contracting States: **CH DE FR GB IT LI NL**

(71) Applicant: **BANYU PHARMACEUTICAL CO., LTD.**
**2-3, Nihonbashi Honcho 2-chome**
**Chuo-ku Tokyo 103(JP)**

(72) Inventor: **OKURA, Akira, Banyu Pharmaceutical Co., Ltd.**
**Exploratory Res. Lab., 9-3, Shimomeguro 2-chome**
**Meguro-ku, Tokyo 153(JP)**
Inventor: **YOSHINARI, Tomoko, Banyu Pharmaceutical Co., Ltd.**
**Exploratory Res. Lab., 9-3, Shimomeguro 2-chome**
**Meguro-ku, Tokyo 153(JP)**
Inventor: **NAKAGAWA, Susumu, Banyu Pharmaceutical Co., Ltd.**
**Okazaki Res. Lab., 9-1, Kamimutsuna 3-chome**
**Okazaki-shi, Aichi 444(JP)**
Inventor: **MANO, Eiichi, Banyu Pharmaceutical Co., Ltd.**
**Okazaki Res. Lab., 9-1, Kamimutsuna 3-chome**
**Okazaki-shi, Aichi 444(JP)**
Inventor: **ARAKAWA, Hiroharu, Banyu Pharmaceutical Co., Ltd.**
**Exploratory Res. Lab., 9-3, Shimomeguro 2-chome**
**Meguro-ku, Tokyo 153(JP)**
Inventor: **USHIJIMA, Ryosuke, Banyu Pharmaceutical Co., Ltd.**
**Okazaki Res. Lab., 9-1, Kamimutsuna 3-chome**
**Okazaki-shi, Aichi 444(JP)**

(74) Representative: **Casalonga, Axel et al**
**BUREAU D.A. CASALONGA - JOSSE**
**Morassistrasse 8**
**W-8000 München 5(DE)**

## (54) PYRIDONECARBOXYLIC ACID DERIVATIVE.

(57) A compound represented by general formula (I), a pharmaceutically acceptable salt of ester thereof, a process for the production thereof, and an antitumor agent containing the same as the active ingredient. In formula (I), $R^1$ represents linear, branched or cyclic lower alkyl, or optionally fluorinated phenyl; $R^2$ and $R^3$ represent each hydrogen, or linear, branched or cyclic lower alkyl; $R^4$ represents hydrogen or $N(R^5)R^6$, wherein $R^5$ and $R^6$ represent each hydrogen, or linear, branched or cyclic lower alkyl; X represents nitrogen or C-Y, wherein Y represents hydrogen, halogen, methyl or methoxy; m and n represent each an integer of 0 or 1; and ⎓ represents a single or double bond.

EP 0 516 861 A1

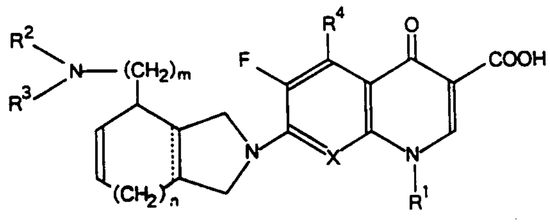

[I]

## Technical Field

This invention relates to novel pyridonecarboxylic acid derivatives, a process for preparation thereof and an antitumor agent containing such a derivative as an effective ingredient.

## Background Art

Heretofore, as chemotherapeutics various derivatives such as antitumor antibiotics, antimetabolites, alkaloids, platinum complexes and epipodophyllotoxins have been researched and developed. Further, although scores of carcinostatics are clinically used, it is the actual situation that sufficient therapeutic results cannot generally be attained because of their side effects or because cancers under treatment gain resistance to carcinostatics.

It is known that a certain kind of quinolonecarboxylic acids have an antibacterial activity, and, for example, norfloxacin, ofloxacin, ciprofloxacin, etc. are clinically widely used as synthetic antibacterial agents. The action mechanism of the antibacterial activity of the quinolonecarboxylic acids is inhibition of the DNA topoisomerase ( gyrase ) of procaryotic cells. The DNA topoisomerase I and DNA topoisomerase II of eucaryotic cells exist in the nuclei of the cells, bind to the DNAs, and change their topology. This action is indispensable for the replication, recombination and transcription of DNA necessary for proliferation of the cells, and thus inhibitors of this enzyme are expected to have an antitumor activity. DNA topoisomerase inhibitors such as etoposide and mitoxanthrone are clinically used as carcinostatics.

Possibility of quinolonecarboxylic acids to exhibit an antitumor effect due to inhibition of the DNA topoisomerase of cancer cells has so far been considered and studied. As a result, it was found that in the past nalidixic acid and in recent years norfloxacin, ofloxacin, ciprofloxacin, etc. inhibit DNA topoisomerase to some degree. However, their actions are far weaker than those of antitumor DNA topoisomerase inhibitors now being put to practical use, and further their antitumor actions are not observed. Thus, the use of quinolonecarboxylic acids as an antitumor agent has not so far been known.

## Disclosure of Invention

The present inventors widely and intensely studied on an activity of various quinolonecarboxylic acid derivatives to inhibit DNA topoisomerase II and an activity thereof to inhibit proliferation of tumor cells. As a result, they found that the compounds of the general formula [ I ] and their pharmaceutically acceptable salts or esters have a very strong DNA topoisomerase II inhibition activity and a very strong tumor cell proliferation inhibition activity. The present inventors further found that the compounds of this invention exhibit, against cancer cells which came to acquire multiple drug resistance, an effect equal to that against drug - sensitive cancer cells, exhibit a therapeutic effect on laboratory mice with transplanted cancer cells by parenteral administration and oral administration, are excellent in tolerance on animals and have extremely high stability, and thus are an utterly novel type of antitumor substances, and completed this invention.

This invention relates to compounds represented by the general formula

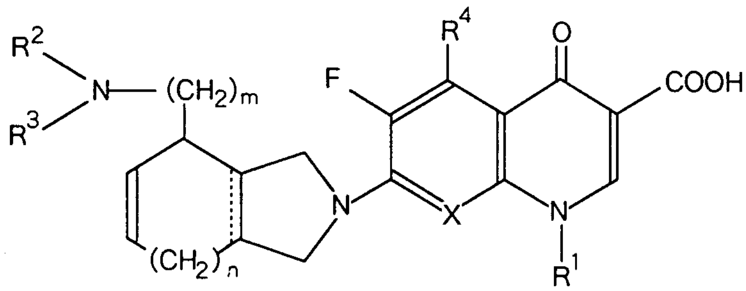

[ I ]

wherein $R^1$ represents a straight - chain, branched or cyclic lower alkyl group or a phenyl group optionally substituted by fluorine atom(s), $R^2$ and $R^3$ each represent a hydrogen atom or a straight - chain, branched or cyclic lower alkyl group, $R^4$ represents a hydrogen atom or - N ($R^5$ ) $R^6$ wherein $R^5$ and $R^6$ each represent a hydrogen atom or a straight - chain, branched or cyclic lower alkyl group, X represents a nitrogen atom or C - Y wherein Y represents a hydrogen atom, halogen atom, methyl group or methoxy group, m and n each represent an integer of 0 or 1, and ⩦ represents a single bond or double bond, or

their pharmaceutically acceptable salts or esters, a process for preparation thereof and their use.

Description is made on symbols and terms disclosed in this description and preferred compounds.

Straight - chain lower alkyl groups mean straight - chain alkyl groups having 1 to 6 carbon atoms, and include, for example, a methyl group, an ethyl group, a propyl group, a butyl group, etc., and a methyl group, an ethyl group, etc. are preferred.

Branched lower alkyl groups mean branched alkyl groups having 3 to 6 carbon atoms, and include, for example, an isopropyl group, an isobutyl group, a tert - butyl group, etc., and preferred among them are a tert - butyl group, etc.

Cyclic lower alkyl groups mean cyclic alkyl groups having 3 to 6 carbon atoms, and include, for example, a cyclopropyl group, a cyclobutyl group, a cyclopentyl group, etc., preferred are a cyclopropyl group, a cyclobutyl group, etc. and particularly preferred is a cyclopropyl group.

Phenyl groups optionally substituted by fluorine atom(s) include, for example, a phenyl group, a 2 - fluorophenyl group, a 3 - fluorophenyl group, a 4 - fluorophenyl group, a 2,3 - difluorophenyl group, a 2, 4 - difluorophenyl group, a 3, 4 - difluorophenyl group, etc., and preferred among them are a 2 - fluorophenyl group, a 2, 4 - difluorophenyl group, a 4 - fluorophenyl group, etc.

Halogen atoms include, for example, a fluorine atom, a chlorine atom, a bromine atom, etc. and preferred among them are a fluorine atom, etc.

Eliminable groups include, for example, halogen atoms such as a fluorine atom, a chlorine atom and a bromine atom, and preferred among them are a fluorine atom, etc.

Protective groups for a carboxyl group include lower alkyl groups such as, for example, a methyl group, an ethyl group, a propyl group, an isopropyl group and a tert - butyl group ; lower alkanoyloxyalkyl groups such as, for example, an acetoxymethyl group , a propionyloxymethyl group , a pivaloyloxymethyl group, a 1 - acetoxyethyl group and a 1 - propionyloxyethyl group; lower alkoxycarbonyloxyalkyl groups such as, for example, a 1 - (methoxycarbonyloxy) ethyl group, a 1 - ( ethoxycarbonyloxy ) ethyl group and a 1 - (isopropoxycarbonyloxy) ethyl group ; (5 - substituted - 2 - oxo - 1,3 - dioxol - 4 - yl) methyl groups such as, for example, a (5 - methyl - 2 - oxo - 1, 3 - dioxol - 4 - yl) methyl group ; lower alkylsilyl groups such as, for example, a trimethylsilyl group and a tert - butyldimethylsilyl group ; for example, an indanyl group, a phthalidyl group, a methoxymethyl group, etc., and particularly preferred are a methyl group, an ethyl group, etc.

Protective groups for an amino group include aralkyl groups such as, for example, a benzyl group, a p - methoxybenzyl group, a 3, 4 - dimethoxybenzyl group, an o - nitrobenzyl group, a p - nitrobenzyl group, a benzhydryl group and a bis (p - methoxyphenyl) methyl group ; lower alkanoyl groups such as, for example, a formyl group, an acetyl group, a propionyl group, a butyryl group, an oxalyl group, a succinyl group and a pivaloyl group ; halo - substituted lower alkanoyl groups such as, for example, a chloroacetyl group, a dichloroacetyl group, a trichloroacetyl group and a trifluoroacetyl group ; arylalkanoyl groups such as, for example, a phenylacetyl group and a phenoxyacetyl group ; lower alkoxycarbonyl groups such as, for example, a methoxycarbonyl group, an ethoxycarbonyl group, a propoxycarbonyl group and a tert - butoxycarbonyl group ; halo - substituted lower alkoxycarbonyl groups such as, for example, a 2 - iodoethyloxycarbonyl group and a 2 , 2 , 2 - trichloroethoxycarbonyl group ; alkenyloxycarbonyl groups such as a 2 - propenyloxycarbonyl group , a 2 - chloro - 2 - propenyloxycarbonyl group , a 3 - methoxycarbonyl - 2 - propenyloxycarbonyl group, a 2 - methyl - 2 - propenyloxycarbonyl group, a 2 - butenyloxycarbonyl group and a cinnamyloxycarbonyl group ; aralkyloxycarbonyl groups such as a benzyloxycarbonyl group , an o - nitrobenzyloxycarbonyl group , a p - nitrobenzyloxycarbonyl group and a phenethyloxycarbonyl group ; and lower alkylsilyl groups such as, for example, a trimethylsilyl group and a tert - butyldimethylsilyl group, and particularly, a 2 - propenyloxycarbonyl group, a tert - butoxycarbonyl group, a p - nitrobenzyloxycarbonyl group, etc. are preferred.

$R^2$ and $R^3$ ($R^{20}$ and $R^{30}$) may be the same or different, and each represent a hydrogen atom or the above - mentioned straight - chain, branched or cyclic lower alkyl group.

Preferred is either the case where both $R^2$ and $R^3$ are hydrogen atoms or the case where $R^3$ is a hydrogen atom and $R^2$ is the aforesaid straight - chain, branched or cyclic lower alkyl group.

$R^4$ represents a hydrogen atom or - N ($R^5$) $R^6$. $R^5$ and $R^6$ ($R^{50}$ and $R^{60}$) may be the same or different, and each represent a hydrogen atom or the aforesaid straight - chain, branched or cyclic lower alkyl group.

As $R^4$, there can be mentioned a hydrogen atom, an amino group, an N - methylamino group, an N - ethylamino group, an N - propylamino group, an N - cyclopropylamino group, an N, N - dimethylamino group, etc., and preferred among them are a hydrogen atom, an amino group, etc.

The compounds of the general formula [ I ] can be converted to pharmaceutically acceptable nontoxic salts or esters thereof according to a conventionl method.

Nontoxic salts of the compounds of the general formula [ I ] mean pharmaceutically acceptable

conventional ones, and can include salts either on the carboxyl group at the 3 - position of the quinoline skeleton or 1, 8 - naphthyridine skeleton or on the base of a free amino group in the side chain at the 7 - position thereof.

Basic addition salts on the carboxyl group include alkali metal salts such as, for example, sodium salts and potassium salts ; alkaline earth metal salts such as, for example, calcium salts and magnesium salts ; for example, ammonium salts ; aliphatic amine salts such as, for example, trimethylamine salts, triethylamine salts, dicyclohexylamine salts, ethanolamine salts, diethanolamine salts, triethanolamine salts and procaine salts ; aralkylamine salts such as , for example , N , N ' - dibenzylethylenediamine salts ; heterocyclic aromatic amine salts such as, for example, pyridine salts, picoline salts, quinoline salts and isoquinoline salts ; quaternary ammonium salts such as, for example, tetramethylammonium salts, tetraethylammonium salts, benzyltrimethylammonium salts, benzyltriethylammonium salts, benzyltributylammonium salts, methyltrioctylammonium salts and tetrabutylammonium salts ; basic amino acid salts such as arginine salts and lysine salts ; etc.

Acid addition salts on the base of a free amino group in the side chain at the 7 - position include inorganic acid salts such as, for example, hydrochlorides, sulfates, nitrates, phosphates, carbonates, bicarbonates and perchlorates ; organic acid salts such as, for example, acetates, propionates, lactates, maleates, fumarates, tartrates, malates, citrates and ascorbates ; sulfonate salts such as, for example, methanesulfonates, isethionates, benzenesulfonates and p - toluenesulfonates ; acidic amino acid salts such as, for example, aspartates and glutamates ; etc.

p - toluenesulfonates are most preferred among the above nontoxic salts.

Nontoxic esters of the compounds of the general formula [ I ] mean pharmaceutically acceptable and conventional ones on the carboxyl group at the 3 - position of the quinoline skeleton or 1, 8 - naphthyridine skeleton. There can, for example, be mentioned esters with an alkanoyloxymethyl group such as an acetoxymethyl group or a pivaloyloxymethyl group : esters with an alkoxycarbonyloxyalkyl group such as a 1 - (ethoxycarbonyloxy) ethyl group ; esters with a phthalidyl group ; esters with a (5 - substituted - 2 - oxo - 1, 3 - dioxol - 4 - yl) methyl group such as a (5 - methyl - 2 - oxo - 1, 3 - dioxol - 4 - yl) methyl group ; etc.

Optical isomers and stereoisomers exist based on the carbon atom at the 2 - position of the azabicyclo [4. 3. 0] group or azabicyclo [3. 3. 0] group in the side chain at the 7 - position and on the crosslinked carbon atoms, and this invention includes both of these isomers.

Among the compounds of the general formula [ I ], compounds wherein m is o and n is 1 are suitable, and preferred compounds among them are compounds represented by the general formula

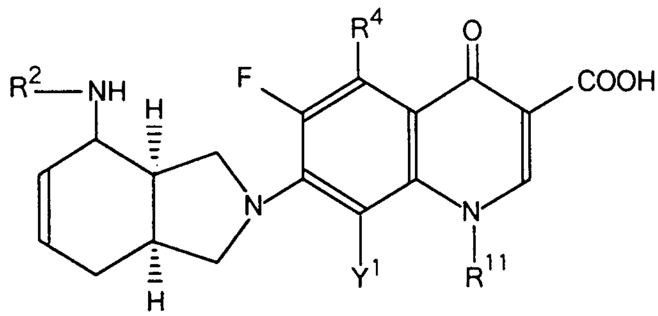

[ I − a]

wherein $R^2$ represents a hydrogen atom or a straight - chain, branched or cyclic lower alkyl group, $R^4$ represents a hydrogen atom or - N ($R^5$) $R^6$ wherein $R^5$ and $R^6$ each represent a hydrogen atom or a straight - chain, branched or cyclic lower alkyl group, $R^{11}$ represents a straight - chain, branched or cyclic lower alkyl group, and $Y^1$ represents a hydrogen atom or a halogen atom, and further, more preferred compounds are compounds represented by the general formula

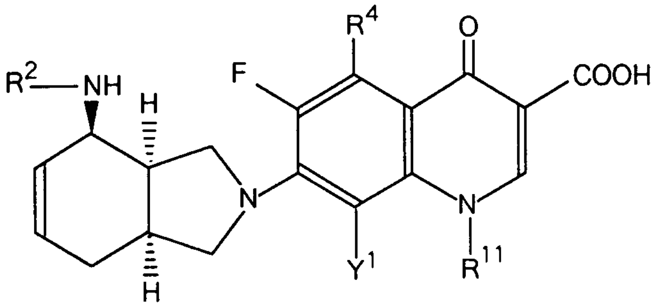

[I − b]

wherein $R^2$, $R^4$, $R^{11}$ and $Y^1$ have the same meanings as above.

Specific examples of the compounds of this invention are set forth in the following tables. The meanings of the abbreviations in the tables are set forth below.

Me : methyl group
Et : ethyl group
Pr : propyl group
iPr : isopropyl group
tBu : tert - butyl group

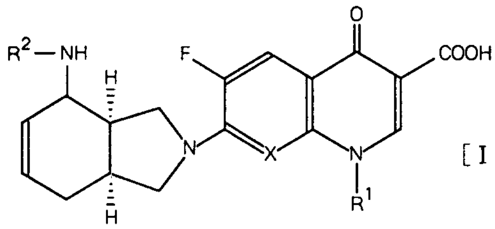

[ I − c]

| Compoud No. | $R^1$ | $R^2$ | X | Compoud No. | $R^1$ | $R^2$ | X |
|---|---|---|---|---|---|---|---|
| 1 | Et | H | CF | 13 | iPr | tBu | CF |
| 2 | Et | Me | CF | 14 | iPr | 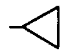 | CF |
| 3 | Et | Et | CF | 15 | tBu | H | CF |
| 4 | Et | Pr | CF | 16 | tBu | Me | CF |
| 5 | Et | iPr | CF | 17 | tBu | Et | CF |
| 6 | Et | tBu | CF | 18 | tBu | Pr | CF |
| 7 | Et | 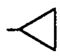 | CF | 19 | tBu | iPr | CF |
| 8 | iPr | H | CF | 20 | tBu | tBu | CF |
| 9 | iPr | Me | CF | 21 | tBu | 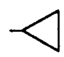 | CF |
| 10 | iPr | Et | CF | 22 | 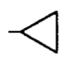 | H | CF |
| 11 | iPr | Pr | CF | 23 | 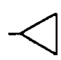 | Me | CF |
| 12 | iPr | iPr | CF | 24 | 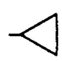 | Et | CF |

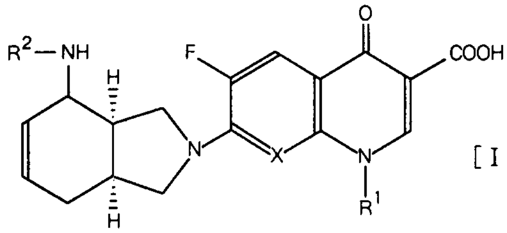

[ I − c]

| Compound No. | $R^1$ | $R^2$ | X | Compound No. | $R^1$ | $R^2$ | X |
|---|---|---|---|---|---|---|---|
| 25 | 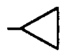 | Pr | CF | 37 | 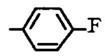 | Et | CF |
| 26 | 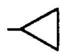 | iPr | CF | 38 | 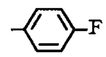 | Pr | CF |
| 27 | 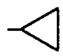 | tBu | CF | 39 | 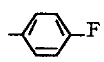 | iPr | CF |
| 28 | 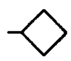 | 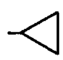 | CF | 40 | 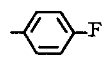 | tBu | CF |
| 29 | 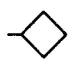 | H | CF | 41 | 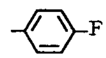 | 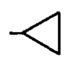 | CF |
| 30 | 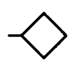 | Me | CF | 42 | 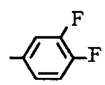 | H | CF |
| 31 | 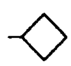 | Et | CF | 43 | 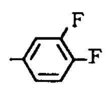 | Me | CF |
| 32 | 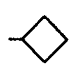 | Pr | CF | 44 | 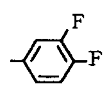 | Et | CF |
| 33 | 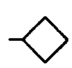 | iPr | CF | 45 | 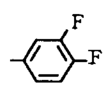 | Pr | CF |
| 34 | 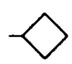 | tBu | CF | 46 | 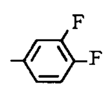 | iPr | CF |
| 35 | 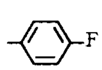 | H | CF | 47 | 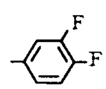 | tBu | CF |
| 36 | 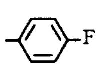 | Me | CF | 48 | Et | H | CH |

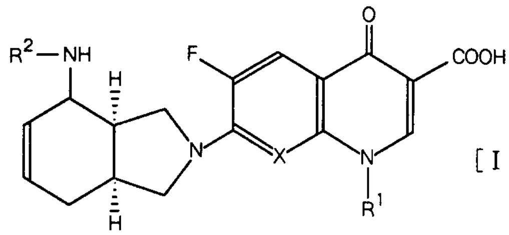

[I - c]

| Compound No. | $R^1$ | $R^2$ | X | Compound No. | $R^1$ | $R^2$ | X |
|---|---|---|---|---|---|---|---|
| 49 | Et | Me | CH | 61 | iPr | 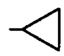 | CH |
| 50 | Et | Et | CH | 62 | tBu | H | CH |
| 51 | Et | Pr | CH | 63 | tBu | Me | CH |
| 52 | Et | iPr | CH | 64 | tBu | Et | CH |
| 53 | Et | tBu | CH | 65 | tBu | Pr | CH |
| 54 | Et | 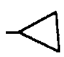 | CH | 66 | tBu | iPr | CH |
| 55 | iPr | H | CH | 67 | tBu | tBu | CH |
| 56 | iPr | Me | CH | 68 | tBu | 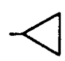 | CH |
| 57 | iPr | Et | CH | 69 | 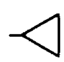 | H | CH |
| 58 | iPr | Pr | CH | 70 | 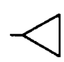 | Me | CH |
| 59 | iPr | iPr | CH | 71 | 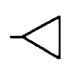 | Et | CH |
| 60 | iPr | tBu | CH | 72 | 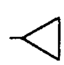 | Pr | CH |

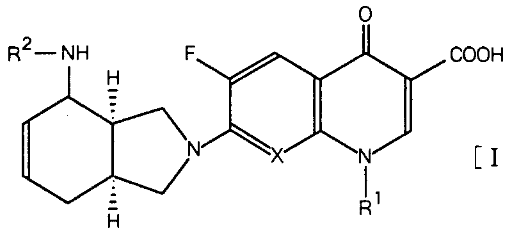

[I - c]

| Compound No. | $R^1$ | $R^2$ | X | Compound No. | $R^1$ | $R^2$ | X |
|---|---|---|---|---|---|---|---|
| 73 | 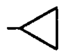 | iPr | CH | 85 | 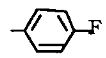 | Pr | CH |
| 74 | 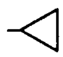 | tBu | CH | 86 | 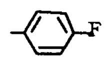 | iPr | CH |
| 75 | 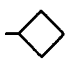 | 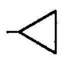 | CH | 87 | 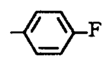 | tBu | CH |
| 76 | 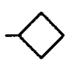 | H | CH | 88 | 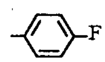 | 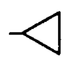 | CH |
| 77 | 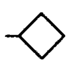 | Me | CH | 89 | 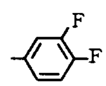 | H | CH |
| 78 | 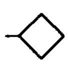 | Et | CH | 90 | 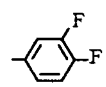 | Me | CH |
| 79 | 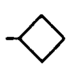 | Pr | CH | 91 | 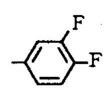 | Et | CH |
| 80 | 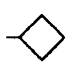 | iPr | CH | 92 | 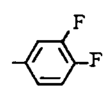 | Pr | CH |
| 81 | 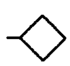 | tBu | CH | 93 | 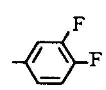 | iPr | CH |
| 82 | 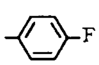 | H | CH | 94 | 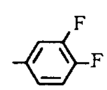 | tBu | CH |
| 83 | 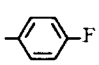 | Me | CH | 95 | 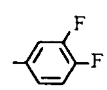 | 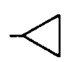 | CH |
| 84 | 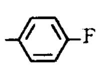 | Et | CH | 96 | Et | H | CMe |

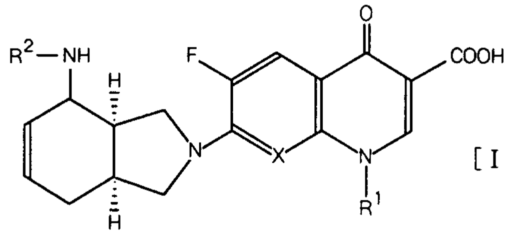

[I - c]

| Compound No. | $R^1$ | $R^2$ | X | Compound No. | $R^1$ | $R^2$ | X |
|---|---|---|---|---|---|---|---|
| 97 | Et | Me | CMe | 109 | iPr | 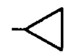 | CMe |
| 98 | Et | Et | CMe | 110 | tBu | H | CMe |
| 99 | Et | Pr | CMe | 111 | tBu | Me | CMe |
| 100 | Et | iPr | CMe | 112 | tBu | Et | CMe |
| 101 | Et | tBu | CMe | 113 | tBu | Pr | CMe |
| 102 | Et | 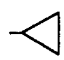 | CMe | 114 | tBu | iPr | CMe |
| 103 | iPr | H | CMe | 115 | tBu | tBu | CMe |
| 104 | iPr | Me | CMe | 116 | tBu | 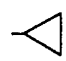 | CMe |
| 105 | iPr | Et | CMe | 117 | 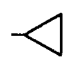 | H | CMe |
| 106 | iPr | Pr | CMe | 118 | 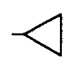 | Me | CMe |
| 107 | iPr | iPr | CMe | 119 | 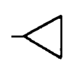 | Et | CMe |
| 108 | iPr | tBu | CMe | 120 | 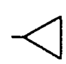 | Pr | CMe |

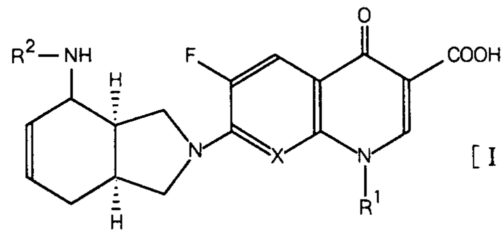

[I – c]

| Compound No. | $R^1$ | $R^2$ | X | Compound No. | $R^1$ | $R^2$ | X |
|---|---|---|---|---|---|---|---|
| 121 | 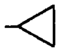 | iPr | CMe | 133 | 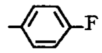 | Pr | CMe |
| 122 | 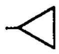 | tBu | CMe | 134 | 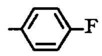 | iPr | CMe |
| 123 | 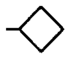 | 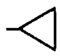 | CMe | 135 | 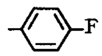 | tBu | CMe |
| 124 | 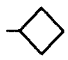 | H | CMe | 136 | 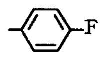 | 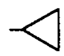 | CMe |
| 125 | 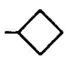 | Me | CMe | 137 | 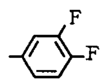 | H | CMe |
| 126 | 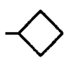 | Et | CMe | 138 | 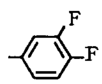 | Me | CMe |
| 127 | 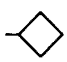 | Pr | CMe | 139 | 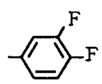 | Et | CMe |
| 128 | 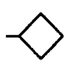 | iPr | CMe | 140 | 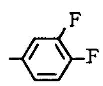 | Pr | CMe |
| 129 | 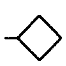 | tBu | CMe | 141 | 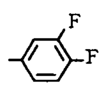 | iPr | CMe |
| 130 | 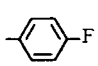 | H | CMe | 142 | 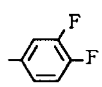 | tBu | CMe |
| 131 | 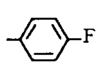 | Me | CMe | 143 | 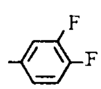 | 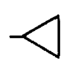 | CMe |
| 132 | 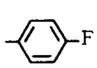 | Et | CMe | 144 | Et | H | C-OMe |

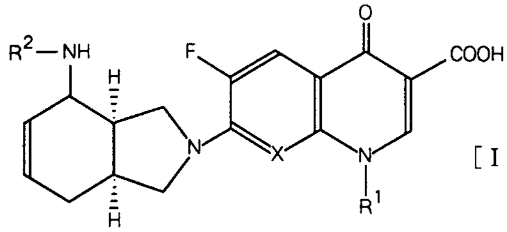

[I - c]

| Compound No. | R¹ | R² | X | Compound No. | R¹ | R² | X |
|---|---|---|---|---|---|---|---|
| 145 | Et | Me | C-OMe | 157 | iPr | 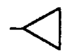 | C-OMe |
| 146 | Et | Et | C-OMe | 158 | tBu | H | C-OMe |
| 147 | Et | Pr | C-OMe | 159 | tBu | Me | C-OMe |
| 148 | Et | iPr | C-OMe | 160 | tBu | Et | C-OMe |
| 149 | Et | tBu | C-OMe | 161 | tBu | Pr | C-OMe |
| 150 | Et | 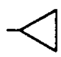 | C-OMe | 162 | tBu | iPr | C-OMe |
| 151 | iPr | H | C-OMe | 163 | tBu | tBu | C-OMe |
| 152 | iPr | Me | C-OMe | 164 | tBu | 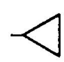 | C-OMe |
| 153 | iPr | Et | C-OMe | 165 | 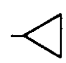 | H | C-OMe |
| 154 | iPr | Pr | C-OMe | 166 | 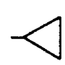 | Me | C-OMe |
| 155 | iPr | iPr | C-OMe | 167 | 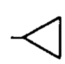 | Et | C-OMe |
| 156 | iPr | tBu | C-OMe | 168 | 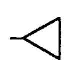 | Pr | C-OMe |

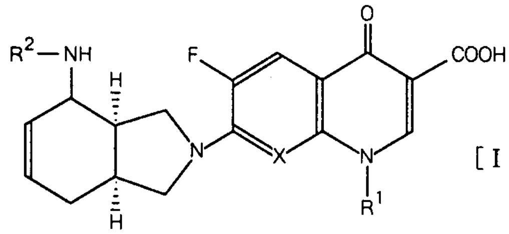

[I − c]

| Compound No. | $R^1$ | $R^2$ | X | Compound No. | $R^1$ | $R^2$ | X |
|---|---|---|---|---|---|---|---|
| 169 | 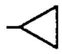 | iPr | C-OMe | 181 | 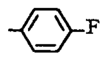 | Pr | C-OMe |
| 170 | 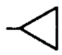 | tBu | C-OMe | 182 | 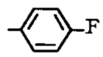 | iPr | C-OMe |
| 171 | 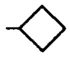 | 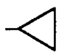 | C-OMe | 183 | 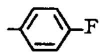 | tBu | C-OMe |
| 172 | 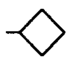 | H | C-OMe | 184 | 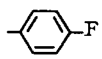 | 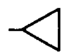 | C-OMe |
| 173 | 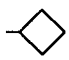 | Me | C-OMe | 185 | 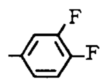 | H | C-OMe |
| 174 | 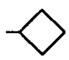 | Et | C-OMe | 186 | 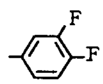 | Me | C-OMe |
| 175 | 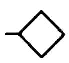 | Pr | C-OMe | 187 | 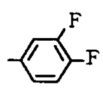 | Et | C-OMe |
| 176 | 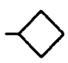 | iPr | C-OMe | 188 | 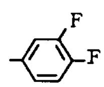 | Pr | C-OMe |
| 177 | 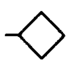 | tBu | C-OMe | 189 | 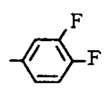 | iPr | C-OMe |
| 178 | 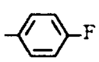 | H | C-OMe | 190 | 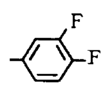 | tBu | C-OMe |
| 179 | 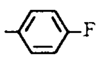 | Me | C-OMe | 191 | 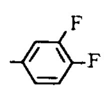 | 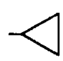 | C-OMe |
| 180iPr | 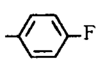 | Et | C-OMe | 192 | Et | H | N |

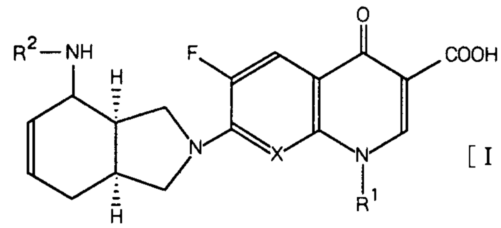

[ I − c]

| Compound No. | $R^1$ | $R^2$ | X | Compound No. | $R^1$ | $R^2$ | X |
|---|---|---|---|---|---|---|---|
| 193 | Et | Me | N | 205 | iPr | 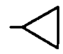 | N |
| 194 | Et | Et | N | 206 | tBu | H | N |
| 195 | Et | Pr | N | 207 | tBu | Me | N |
| 196 | Et | iPr | N | 208 | tBu | Et | N |
| 197 | Et | tBu | N | 209 | tBu | Pr | N |
| 198 | Et | 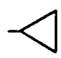 | N | 210 | tBu | iPr | N |
| 199 | iPr | H | N | 211 | tBu | tBu | N |
| 200 | iPr | Me | N | 212 | tBu | 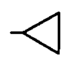 | N |
| 201 | iPr | Et | N | 213 | 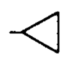 | H | N |
| 202 | iPr | Pr | N | 214 | 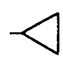 | Me | N |
| 203 | iPr | iPr | N | 215 | 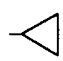 | Et | N |
| 204 | iPr | tBu | N | 216 | 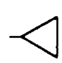 | Pr | N |

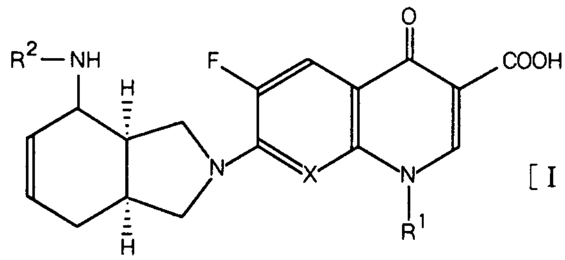

[I − c]

| Compound No. | $R^1$ | $R^2$ | X | Compound No. | $R^1$ | $R^2$ | X |
|---|---|---|---|---|---|---|---|
| 217 | 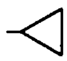 | iPr | N | 229 | 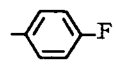 | Pr | N |
| 218 | 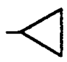 | tBu | N | 230 | 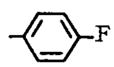 | iPr | N |
| 219 | 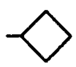 | 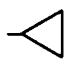 | N | 231 | 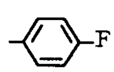 | tBu | N |
| 220 | 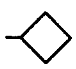 | H | N | 232 | 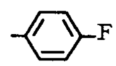 | 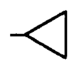 | N |
| 221 | 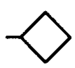 | Me | N | 233 | 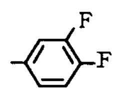 | H | N |
| 222 | 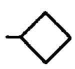 | Et | N | 234 | 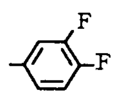 | Me | N |
| 223 | 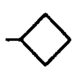 | Pr | N | 235 | 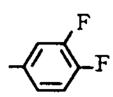 | Et | N |
| 224 | 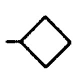 | iPr | N | 236 | 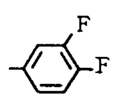 | Pr | N |
| 225 | 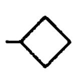 | tBu | N | 237 | 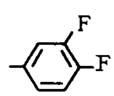 | iPr | N |
| 226 | 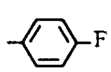 | H | N | 238 | 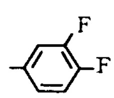 | tBu | N |
| 227 | 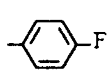 | Me | N | 239 | 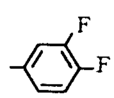 | 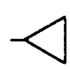 | N |
| 228 | 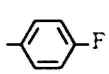 | Et | N | 240 | 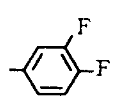 | H | CF |

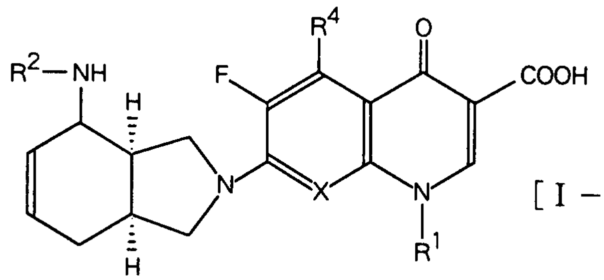

[I - d]

| Compound No. | $R^1$ | $R^2$ | $R^4$ | X | Compound No. | $R^1$ | $R^2$ | $R^4$ | X |
|---|---|---|---|---|---|---|---|---|---|
| 241 | Et | H | $NH_2$ | CF | 247 | 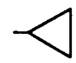 | H | NHMe | CF |
| 242 | tBu | H | $NH_2$ | CF | 248 | 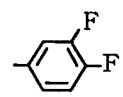 | H | NHMe | CF |
| 243 | 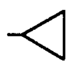 | H | $NH_2$ | CF | 249 | Et | H | $NMe_2$ | CF |
| 244 | 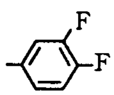 | H | $NH_2$ | CF | 250 | tBu | H | $NMe_2$ | CF |
| 245 | Et | H | NHMe | CF | 251 | 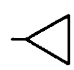 | H | $NMe_2$ | CF |
| 246 | tBu | H | NHMe | CF | 252 | 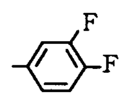 | H | $NMe_2$ | CF |

Preferred compounds among the above compounds are, for example,

7- (1, 6 - cis - 2 - amino - 8 - azabicyclo [4. 3. 0] non - 3 - en - 8- yl) - 1 - ethyl - 6, 8 - difluoro - 1, 4 - dihydro - 4 - oxo - 3 - quinolinecarboxylic acid (compound 1),

7 - (1, 6 - cis - 2 - amino - 8 - azabicyclo [4. 3. 0] non - 3 - en - 8 - yl) - 1 - tert - butyl - 6, 8 - difluoro - 1, 4 - dihydro - 4 - oxo - 3 - quinolinecarboxylic acid (compound 15),

7- (1, 6 - cis - 2 - amino - 8 - azabicyclo [4. 3. 0] non - 3 - en - 8 - yl) - 1 - cyclopropyl - 6, 8 - difluoro - 1, 4 - dihydro - 4 - oxo - 3 - quinolinecarboxylic acid (compound 22),

1 - cyclopropyl - 6, 8 - difluoro - 1, 4 - dihydro - 7 - [1, 6 - cis - 2 - (N - methylamino) - 8 - azabicyclo [4. 3. 0] non - 3 - en - 8 - yl] - 4 - oxo - 3 - quinolinecarboxylic acid (compound 23),

7- (1, 6 - cis - 2 -amino- 8 - azabicyclo [4. 3. 0] non - 3 - en - 8 - yl) -6, 8 - difluoro - 1 - ( 4 - fluoro) phenyl - 1, 4 - dihydro - 4 - oxo - 3 - quinolinecarboxylic acid (compound 35),

7 - (1, 6 - cis - 2 - amino - 8 - azabicyclo [4. 3. 0] non - 3 - en - 8- yl) - 6, 8 - difluoro - 1 - ( 3, 4 - difluoro) phenyl - 1, 4 - dihydro - 4 - oxo - 3 - quinolinecarboxylic acid (compound 42),

7 - (1, 6 - cis - 2 - amino - 8 - azabicyclo [4. 3. 0] non - 3 - en - 8 - yl) - 1 - ethyl - 6 - fluoro - 1, 4 - dihydro - 4 - oxo - 3 - quinolinecarboxylic acid (compound 48),

7 - (1, 6 - cis - 2 - amino - 8 - azabicyclo [4. 3. 0] non - 3 - en - 8 - yl) - 1 - tert - butyl - 6 - fluoro - 1, 4 - dihydro - 4 - oxo - 3 - quinolinecarboxylic acid (compound 62),

7 - (1, 6 - cis - 2 - amino - 8 - azabicyclo [4. 3. 0] non - 3 - en - 8 - yl) - 1 - cyclopropyl - 6 - fluoro - 1, 4 - dihydro - 4 - oxo - 3 - quinolinecarboxylic acid (compound 69),

7 - (1, 6 - cis - 2 - amino - 8 - azabicyclo [4. 3. 0] non - 3 - en - 8 - yl) - 6 - fluoro - 1 - (4 - fluoro) phenyl - 1, 4 - dihydro - 4 - oxo - 3 - quinolinecarboxylic acid (compound 82),

7 - (1, 6 - cis - 2 - amino - 8 - azabicyclo [4. 3. 0] non - 3 - en - 8 - yl) - 6 - fluoro - 1 - (3, 4 - difluoro) phenyl - 1, 4 - dihydro - 4 - oxo - 3 - quinolinecarboxylic acid (compound 89),

7- (1, 6 - cis - 2 - amino - 8 - azabicyclo [4. 3. 0] non - 3 - en - 8 - yl) - 1 - ethyl - 6 - fluoro - 1, 4 - dihydro - 4 - oxo - 3 - quinolinecarboxylic acid (compound 96),

7 - (1, 6 - cis - 2 - amino - 8 - azabicyclo [4. 3. 0] non - 3 - en - 8 - yl) - 1 - tert - butyl - 6 - fluoro - 1, 4 - dihydro - 8 - methyl - 4 - oxo - 3 - quinolinecarboxylic acid (compound 110),

7 - (1, 6 - cis- 2 - amino - 8 - azabicyclo [4. 3. 0] non - 3 - en - 8 - yl) - 1 - cyclopropyl - 6 - fluoro - 1, 4 - dihydro - 8 - methyl - 4 - oxo - 3 - quinolinecarboxylic acid (compound 117),

7 - (1, 6 - cis - 2 - amino - 8 - azabicyclo [4. 3. 0] non - 3 - en - 8 - yl) - 6 - fluoro - 1 - (4 - fluoro) phenyl - 1, 4 - dihydro - 8 - methyl - 4 - oxo - 3 - quinolinecarboxylic acid (compound 130),

7 - (1, 6 - cis - 2 - amino - 8 - azabicyclo [4. 3. 0] non - 3 - en - 8 - yl) - 6 - fluoro - 1 - (3, 4 - difluoro) phenyl - 1, 4 - dihydro - 8 - methyl - 4 - oxo - 3 - quinolinecarboxylic acid (compound 137),

7 - (1, 6 - cis - 2 - amino - 8 - azabicyclo [4. 3. 0] non - 3 - en - 8 - yl) - 1 - ethyl - 6 - fluoro - 1, 4 - dihydro - 8 - methoxy - 4 - oxo - 3 - quinolinecarboxylic acid (compound 144),

7 - (1, 6 - cis - 2 - amino - 8 - azabicyclo [4. 3. 0] non - 3 - en - 8 - yl) - 1 - tert - butyl - 6 - fluoro - 1, 4 - dihydro - 8 - methoxy - 4 - oxo - 3 - quinolinecarboxylic acid (compound 158),

7 - (1, 6 - cis - 2 - amino - 8 - azabicyclo [4. 3. 0] non - 3 - en - 8 - yl) - 1 - cyclopropyl - 6 - fluoro - 1, 4 - dihydro - 8 - methoxy - 4 - oxo - 3 - quinolinecarboxylic acid (compound 165),

7- (1, 6 - cis - 2 - amino - 8 - azabicyclo [4. 3. 0] non - 3 - en - 8 - yl) - 6 - fluoro - 1 - (4 - fluoro) phenyl - 1, 4 - dihydro - 8 - methoxy - 4 - oxo - 3 - quinolinecarboxylic acid (compound 178),

7 - (1, 6 - cis - 2 - amino - 8 - azabicyclo [4. 3. 0] non - 3 - en - 8 - yl) - 6 - fluoro - 1 - (3, 4 - difluoro) phenyl - 1, 4 - dihydro - 8 - methoxy - 4 - oxo - 3 - quinolinecarboxylic acid (compound 185),

7 - (1, 6 - cis - 2 - amino - 8 - azabicyclo [4. 3. 0] non - 3 - en - 8 - yl) - 1 - ethyl - 6 - fluoro - 1, 4 - dihydro - 4 - oxo - 1, 8 - naphthyridine - 3 - carboxylic acid (compound 192),

7 - (1, 6 - cis - 2 - amino - 8 - azabicyclo [4. 3. 0] non - 3 - en - 8 - yl) - 1 - tert - butyl - 6 - fluoro - 1, 4 - dihydro - 4 - oxo - 1, 8 - naphthyridine - 3 - carboxylic acid (compound 206),

7 - (1, 6 - cis - 2 - amino - 8 - azabicyclo [4. 3. 0] non - 3 - en - 8 - yl) - 1 - cyclopropyl - 6 - fluoro- 1, 4 - dihydro - 4 - oxo - 1, 8 - naphthyridine - 3 - carboxylic acid (compound 213),

7 - (1, 6 - cis- 2 - amino - 8 - azabicyclo [4. 3. 0] non - 3 - en - 8 - yl) - 6 - fluoro - 1 - (4 - fluoro) phenyl - 1, 4 - dihydro - 4 - oxo - 1, 8 - naphthyridine - 3 - carboxylic acid (compound 226),

7 - (1, 6 - cis - 2 - amino - 8 - azabicyclo [4. 3. 0] non - 3 - en - 8 - yl) - 6 - fluoro - 1 - (3, 4 - difluoro) phenyl - 1, 4 - dihydro - 4 - oxo - 1, 8 - naphthyridine - 3 - carboxylic acid (compound 233),

7 - (1, 6 - cis - 2 - amino - 8 - azabicyclo [4. 3. 0] non - 3 - en - 8 - yl) - 6, 8 - difluoro - 1 - (2, 4 - difluorophenyl) - 1, 4 - dihydro - 4 - oxo - 3 - quinolinecarboxylic acid (compound 240),

5 - amino- 7- (1, 6 - cis - 2 -amino- 8- azabicyclo [4. 3. 0] non - 3 - en - 8 - yl) - 1 - ethyl - 6, 8 - difluoro - 1, 4 - dihydro - 4 - oxo - 3 - quinolinecarboxylic acid (compound 241),

5 - amino - 7 - (1, 6 - cis - 2 - amino - 8 - azabicyclo [4. 3. 0] non - 3 - en - 8 - yl) - 1 - tert - butyl - 6, 8 - difluoro- 1, 4 - dihydro - 4 - oxo - 3 - quinolinecarboxylic acid (compound 242),

5 - amino - 7 - (1, 6 - cis - 2 - amino - 8 - azabicyclo [4. 3. 0] non - 3 - en - 8 - yl) - 1 - cyclopropyl - 6, 8 - difluoro- 1, 4 - dihydro - 4 - oxo - 3 - quinolinecarboxylic acid (compound 243),

5 - amino - 7 - (1, 6 - cis - 2 - amino - 8 - azabicylo [4. 3. 0] non - 3 - en - 8 - yl) - 6, 8 - difluoro - 1 - (3, 4 - difluoro) phenyl - 1, 4 - dihydro - 4 - oxo - 3 - quinolinecarboxylic acid (compound 244), etc. Among them, particularly preferred are compounds represented by the general formula [ I - b], namely compounds such as, for example,

7 - [(1R*, 2R*, 6S*) - 2 - amino - 8- azabicyclo [4. 3. 0] non - 3 - en - 8 - yl] - 1 - cyclopropyl - 6, 8 - difluoro - 1, 4 - dihydro - 4 - oxo - 3 - quinolinecarboxylic acid,

1 - cyclopropyl - 6, 8 - difluoro- 1, 4 - dihydro - 7 - [(1R*, 2R*, 6S*) - 2 - (N - methylamino) - 8 - azabicyclo [4. 3. 0] non - 3 - en - 8 - yl] - 4 - oxo - 3 - quinolinecarboxylic acid,

7 - [(1R*, 2R*, 6S*) - 2 - amino - 8 - azabicyclo [4. 3. 0] non - 3 - en - 8 - yl] - 1 - cyclopropyl - 6 - fluoro - 1, 4 - dihydro - 4 - oxo - 3 - quinolinecarboxylic acid,

7 - [(1R*, 2R*, 6S*) - 2 - amino - 8 - azabicyclo [4. 3. 0] non - 3 - en - 8 - yl] - 6, 8 - difluoro - 1 - (2, 4 - difluorophenyl) - 1, 4 - dihydro - 4 - oxo - 3 - quinolinecarboxylic acid,

7 - [(1R*, 2R*, 6S*) - 2 - amino - 8 - azabicyclo [4. 3. 0] non - 3 - en - 8 - yl] - 1 - ethyl - 6 - fluoro- 1, 4 - dihydro - 4 - oxo - 3 - quinolinecarboxylic acid,

7 - [(1R*, 2R*, 6S*) - 2 - amino - 8 - azabicyclo [4. 3. 0] non - 3 - en - 8 - yl] - 1 - cyclopropyl - 6 - fluoro - 1, 4 - dihydro - 8 - methoxy - 4 - oxo - 3 - quinolinecarboxylic acid, and

5 - amino - 7 - [(1R*, 2R*, 6S*) - 2 - amino - 8 - azabicyclo [4. 3. 0] non - 3 - en - 8- yl] - 1 - cyclopropyl - 6, 8 - difluoro - 1, 4 - dihydro - 1 - oxo - 3 - quinolinecarboxylic acid.

A process for preparation of the compounds of this invention is described.

A compound of this invention reprsented by the general formula [ I ] can be prepared by reacting, in the

presence of a deacidifying agent, a compound represented by the following general formula or a salt thereof

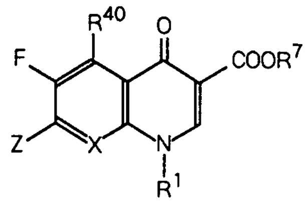

[IV]

wherein $R^1$ represents a straight - chain, branched or cyclic lower alkyl group or a phenyl group optionally substituted by fluorine atom(s), $R^7$ represents a hydrogen atom or a protective group for a carboxyl group, $R^{40}$ represents a hydrogen atom or -N ($R^{50}$) $R^{60}$ wherein $R^{50}$ and $R^{60}$ each represent a hydrogen atom, a protective group for an amino group or a straight - chain, branched or cyclic lower alkyl group, X represents a nitrogen atom or C - Y wherein Y represents a hydrogen atom, halogen atom, methyl group or methoxy group, and Z represents an eliminable group,
with a compound represented by the following formula or a salt thereof

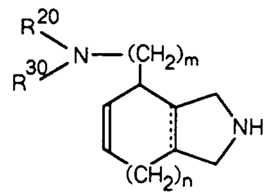

[III]

wherein $R^{20}$ and $R^{30}$ each represent a hydrogen atom, a protective group for an amino group, or a straight - chain, branched or cyclic lower alkyl group, m and n each represent an integer of 0 or 1, and $\cdots\cdots$ represents a single bond or double bond, to give a compound represented by the general formula

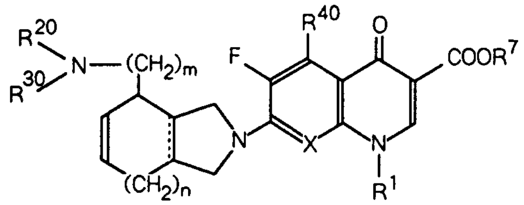

[II]

wherein $R^1$, $R^7$, $R^{20}$, $R^{30}$, $R^{40}$, X, m, n and $\cdots\cdots$ have the same meanings as defined above,
and, if necessary, removing the protective groups of the compound.

The reaction between the compound of the general formula [III] and the compound of the general formula [IV] can be carried out in an inert solvent, usually an alcohol such as, for example, ethanol or methanol ; an ether such as, for example, dioxane or tetrahydrofuran ; an aromatic hydrocarbon such as, for example, benzene or toluene ; for example, acetonitrile ; N, N - dimethylformamide ; dimethyl sulfoxide ; or the like. The reaction temperature may be room temperature to 200 ° C, preferably 80 to 150 ° C, and the reaction time may be 0.5 to 24 hours, usually 1 to 6 hours.

This reaction is usually carried out in the presence of an deacidifying agent using 1.0 to 1.2 mol of the compound of the general formula [III] per mol of the compound of the general formula [IV]. The deacidifying agent is used in a quantity of 1.0 to 1.2 mol per mol of the compound of the general formula [IV]. Deacidifying agents include alkali metal hydroxides such as, for example, sodium hydroxide and potassium hydroxide ; alkali metal carbonates such as, for example, sodium carbonate and potassium carbonate ; alkali metal bicarbonates such as, for example, sodium bicarbonate ; organic bases such as, for example, triethylamine, pyridine, 1, 8 - diazabicyclo [5. 4. 0] undec - 7 - ene (DBU) and 1, 5 - diazabicyclo [4. 3. 0] non - 5 - ene (DBN). The reaction can also be carried out using an excess quantity of a basic compound such as triethylamine or pyridine both as a deacidifying agent and as a solvent.

After completion of the reaction, usual treatments are carried out to obtain a crude product represented by the general formula [II]. When $R^7$ of the compound of the general formula [II] is a protective group for a carboxyl group, the protective group for a carboxyl group can be removed without purification to prepare the compound of the general formula [ I ].

The removal of the protective group can be carried out by hydrolysis or solvolysis in an inert solvent in the presence of an acid or a base according to a conventional method.

Inert solvents include, for example, water, methanol, ethanol, propanol, tetrahydrofuran and a mixed solvent thereof.

Acids used in the reaction include, for example, hydrochloric acid, sulfuric acid, etc., and such an acid is used in a quantity of 2 to 20 mol per mol of the compound of the general formula [II]. The reaction temperature can be 0 to 80 °C and the reaction is completed in 0.5 to 3 hours.

Bases used in the reaction include alkali metal hydroxides such as, for example, sodium hydroxide and potassium hydroxide; alkali metal carbonates such as, for example, sodium carbonate and potassium carbonate ; alkali metal bicarbonates such as, for example, sodium bicarbonate and potassium bicarbonate. The reaction is carried out using 1 to 3 mol of such a base per mol of the compound of the general formula [II], and the reaction temperature can be 0 to 50 °C and the reaction is completed in 5 to 30 minutes.

After completion of the reaction for removal of the protective group, the compound of the general formula [ I ] can be isolated by usual treatment method (s), for example by solvent extraction, crystallization, column chromatography using silica gel or an adsorption resin, or the like. The compound of the general formula [ I ] can be obtained in the form of an inner salt, a nontoxic addition salt or a hydrate, and they can mutually be converted by a conventional method in accordance with the purpose.

When the protective group for the carboxyl group at the 3 - position of the compound of the general formula [II] is a lower alkanoyloxyalkyl group such as , for example , an acetoxymethyl group or pivaloyloxymethyl group ; a methoxymethyl group ; an indanyl group ; a phthalidyl group ; or the like, the resultant ester is physiologically hydrolyzed in the living body, and, therefore, can directly be administered to human beings and animals without removal of the protective group.

Compounds represented by the general formula [III] are novel compounds not disclosed in literatures, and preparation methods for typical compounds are disclosed in Reference example 1 and Reference example 2.

Compounds of the general formula [IV] can be prepared according to a method disclosed, for example in Japanese Published Unexamined Patent Application No. 212,475/1984, 172,981/1985, 267,573/1986, 215,572/1987 or 297,366/1988 or the like.

In order to demonstrate the utility of this invention, the following antitumor activity tests were made using the compounds of Examples 9, 11 and 13 as representative compounds.

## Test example 1

DNA topoisomerase inhibition activity

DNA topoisomerase inhibition activities were measured using etoposide and camptothecin as comparative compounds.

In this experiment, as enzymes were used DNA topoisomerase I and DNA topoisomerase II obtained by purifying an extract from the nuclei of cells of L1210 murine leukemia by column chromatography using hydroxylapatite and phosphocellulose, respectively, and as a DNA was used supercoiled pBR 322 plasmid DNA.

Measurement of inhibition effect was made by the method disclosed in a literature, i. e. Okura et al., Biochem. Biophys. Res. Comm. 157, 183 - 189, 1988, namely by warming at 37 °C for 10 minutes the supercoiled DNA in DNA topoisomerase I or DNA topoisomerase II solutions containing a drug in various concentrations, and then detecting the resultant topology changes of the DNA as changes of the electrophoretic images in agarose - gel electrophoresis.

The results are shown below.

| Inhibition effect against DNA relaxation with DNA topoisomerase | | |
|---|---|---|
| Drug | $IC_{50}$ (μg/ml) | |
| | DNA topoisomerase II | DNA topoisomerase I |
| Compound of Ex.9 | 2 | 200 |
| Compound of Ex.11 | 2 | 1250 |
| Compound of Ex.13 | 4 | 100 |
| Etoposide | 12.5 | 750 |
| Camptothecin | > 10 | 1.2 |

As shown in the above table, all the compounds of Examples 9, 11 and 13 specifically inhibit DNA topoisomerase II, and their actions are much stronger than that of VP - 16, a usual specific inhibitor of DNA topoisomerase II.

Test example 2

In vitro cytotoxicity against L1210 murine leukemia

In vitro cytotoxicities were measured using etoposide and camptothecin as comparative compounds.

In this experiment, respective $2 \times 10^4$ L1210 cells were cultured at 37°C under 5% $CO_2$ gas for 3 days in respective RPMI 1640 media containing a drug in various concentrations and 10 % fetal bovine serum, and the numbers of the cells after the culture were counted using a Coulter counter. The results are shown below.

| Proliferation inhibition effect against L1210 cells | |
|---|---|
| Drug | $IC_{50}$ (ng/ml) |
| Compound of Ex.9 | 19 |
| Compound of Ex.11 | 32 |
| Compound of Ex.13 | 1.5 |
| Etoposide | 21 |
| Camptothecin | 40 |

As shown in the above table, all the compounds used in this invention exhibited a very strong cellular proliferation inhibition effect , and concentrations to inhibit cellular proliferation by 50% ($IC_{50}$) were 100 ng/ml or less. This result suggests the usefulness of the compounds of this invention in use for treatment of cancers.

Test example 3

Proliferation inhibition effect against P388/multiple drug - resistant cells

Proliferation inhibition effects againt P388/multiple drug - resistant cells were measured using vincristine and adriamycin as comparative compounds.

In this experiment, a drug - sensitive strain (P388/S) of a murine leukemia cell P388 and a drug - resistant strain (P388/VCR, T. Turuo, H. Iida, S. Tsukagoshi, et al., Cancer Research 42, 4730 - 4733, 1982) were suspended respectively in RPMI 1640 media containing a drug in various concentrations, 10 % fetal bovine serum and 20 μ M β - mercaptoethanol, respectively, and seeded in a 96 - well microtest plate in a number of $10^4$ cells/well, and cultured at 37°C under $CO_2$ gas for 3 days. Calculation of 50% proliferation inhibition concentration ($IC_{50}$) was made by the MTT method (M. C. Alley, D. A. Scudiero, A. Monks, et al., Cancer Research 48, 589 - 601, 1988). The results are shown below.

| Proliferation inhibition effect against P388/V multiple drug - resistant cells | | |
|---|---|---|
| Drug | $IC_{50}$ (ng/ml) | |
| | P388/V | P388/VCR |
| Compound of Ex.9 | 18 | 18 |
| Vincristine | 0.32 | 19 |
| Adriamycin | 21 | 63 |

As shown in the above table, the compound of Example 9 not only exhibited a very strong proliferation inhibition effect against the drug - sensitive P388 cells (P388/S) but exhibited an equal effect against the multiple drug - resistant P388/V cells, which suggested the clinical usefulness thereof.

Test example 4

Life - span prolonging effect on L1210 and P388 leukemias - bearing mice

Life - span prolonging effects on L1210 and P388 leukemias - bearing mice were measured using etoposide as a comparative compound.

In this experiment, $10^6$ L1210 cells or $10^4$ P388 cells were intraperitoneally transplanted into $CDF_1$ mice (♀, 5 weeks old), a drug in the various concentrations was intraperitoneally injected once a day for continuous 10 days from the day after the transplantation, and survival periods after the tumor transplantation were recorded.

The results are shown below.

Life－span prolonging effect on L1210 and P388 leukemias－bearing mice

| Drug | Dose mg／kg | Number of animals | Mean survival days (T／C%) | |
|---|---|---|---|---|
| | | | L1210 | P388 |
| Physiological saline | 0 | 10 | 13.7 (100) | 11.2 (100) |
| Compound of Ex.9 | 0.020 | 5 | 31.0 (226) | 12.0 (107) |
| | 0.078 | 5 | 32.4 (236) | 14.0 (125) |
| | 0.313 | 5 | 21.2 (155) | 16.4 (146) |
| Compound of Ex.11 | 0.020 | 5 | 25.8 (188) | － |
| | 0.078 | 5 | >50.8 (371) | － |
| | 0.313 | 5 | 16.4 (120) | － |
| Etoposide | 0.020 | 5 | 14.2 (104) | 12.8 (114) |
| | 0.078 | 5 | 22.8 (166) | 16.6 (148) |
| | 0.313 | 5 | 31.4 (229) | 22.2 (198) |

$$* T/C\% = \frac{\text{Mean survival days of the drug administration group}}{\text{Mean survival days of the control (physiological saline) adminstration group}}$$

As apparent from the above table, the compounds of Example 9 and Example 11 were effective at an administration quantity of 0.02 mg/kg at which etoposide exhibited no effect, and had a wide effective dose range (0.02 to 0.313 mg/kg).

Test example 5

Acute toxicity against a mouse

5 mg/kg portions of the compound of Example 9 were intravenously injected into $CDF_1$ mice (♀, 5 weeks old) according to a conventional manner, respectively. No case of death was found in observation for one week.

Therefore, the compounds of this invention are compounds having only small toxicity and useful as antitumor agents.

Further, the compounds of this in vention exhibit good antibacterial activities and are useful as antibacterial agents. In order to demonstrate the usefulness of the compounds of this invention as antibacterial agents, the in vitro antibacterial activities of the compounds of this invention against various bacteria were measured by the following agar plate dilution method [the standard method prescribed by Japan Society of Chemotherapy : Chemotherapy 29, 76 -79 (1981)]. One platinum loopful portions of a test strain (quantity of each strain inoculated : $10^6$ CFU/ml) cultured overnight in Mueller Hinton broth were inoculated respectively into MH agars containing an antibacterial agent in various concentrations. After 16 hours culture at 37° C, minimum inhibitory concentration (MIC: μg/ml) was measured. The results are shown in the following table.

| Minimum inhibitory concentration (MIC ; μg/ml) | | | |
|---|---|---|---|
| Test bacteria | Compound of Ex.1 | Compound of Ex.2 | Compound of Ex.13 |
| S.aureus JS1* | < 0.006 | < 0.006 | < 0.006 |
| S.aureus BB5703 | < 0.006 | < 0.006 | < 0.006 |
| S.aureus BB5769 | < 0.006 | < 0.006 | < 0.006 |

* β - lactamase - producing bacterium

The compounds of this invention have excellent antibacterial activities against various Gram - positive bacteria and Gram - negative bacteria, and are useful antibacterial agents for treatment and prophylaxis of human bacterial infectious diseases caused by these pathogenic bacteria. Representative pathogens sensitive to the antibacterial agents of this invention can include species, for example, of the genera Staphylococcus, Enterococcus, Escherichia, Enterobacter, Klebsiella, Serratia, Proteus, Pseudomonas, etc., and particularly, they exhibited excellent antibacterial activities against methicillin resistant Staphylococcus aureus and thienamycin resistant Pseudomonas aeruginosa.

The compounds of this invention can be used in the forms of pharmaceutical preparations suitable for parenteral administration, oral administration or external administration, wherein they are mixed with carriers, i. e. solid or liquid excipients known in the field. Chief administration is topical administration or parenteral administration by injection (intravenous injection or intramuscular injection). Pharmaceutical preparations include, for example, liquid preparations such as injection, syrups and emulsions ; solid preparations such as tablets, capsules and granules ; external preparations such as ointments and suppositories ; etc. These preparations may, if necessary, contain additives usually used such as a base, an auxiliary, a stabilizer, a wetting agent, an emulsifier, an absorption accelerator and a surfactant.

Examples of such additives are distilled water for injection, Ringer's solution, glucose, sucrose syrup, gelatin, edible oil, cacao butter, ethylene glycol, sugar, corn starch, magnesium stearate, tale, etc.

Dose as an antitumor agent varies depending on symptoms, body weights, ages and the distinction of sex of patients ; dosage forms ; the number of times of dosage ; etc., but, usually, the effective dose of the present compound in case of intravenous injection can be about 1 to 75 mg, preferably 1 to 15 mg per adult per day. Further, the effective dose of the present compound in case of oral administration can be 10 to 1,000mg, preferably 10 to 500 mg per day.

Dose as an antibacterial agent varies depending on symptoms, body weights, ages and the distinction of sex of patients ; dosage forms ; the number of times of dosage ; etc., but, usually, preferred daily dose to an adult is in the range of about 5 to 50 mg/kg as the effective ingredient, and preferred daily dose to a child is in the range of about 5 to 25 mg/kg. Preferably, the agent is administered once a day, or several times a day in a divided form.

Examples and Reference examples

This invention is further specifically described below according to examples and reference examples, but the invention is not limited thereby at all.

As for thin layer chromatography in examples and reference examples, Silicagel $60F_{245}$ (Merck) was used as the plate and a UV dectector or a ninhydrin coloring method was used as a detection means. Wakogel ™ C - 300 (Wako Pure Chemical Industries, Ltd.) was used as the silica gel for a column. In NMR spectrum, in case measurement was made using a heavy dimethyl sulfoxide or heavy chloroform solution, tetramethylsilane (TMS) was used as the internal standard, measurement was made using an XL - 200 (200 MHz ; Varian) type spectrometer, and all the δ values were expressed by ppm.

Meanings of abbreviations in NMR measurement are set forth below.

s : singlet
d : doublet
br : broad
m : multiplet
J : coupling constant
Hz : Hertz
$CDCl_3$ : heavy chloroform
$CD_3OD$ : heavy methanol
TFA - $d_1$ : heavy trifluoroacetic acid

Meanings of abbreviations in reaction formulae are set forth below.

Alloc : allyloxycarbonyl group
Boc - S : 2 - (tert - butoxycarbonylthio) - 4, 6 - dimethylpyrimidine group
Boc : tert - butoxycarbonyl group
Bn : benzyl group
Cbz : benzyloxycarbonyl group
Me : methyl group
TFA : trifluoroacetic acid

Example 1

7 - [(1R*, 2R*, 6S*) - 2 - amino - 8 - azabicyclo [4.3.0] non - 3 - en - 8 - yl] - 1 - cyclopropyl - 6, 8 - difluoro - 1, 4 - dihydro-4 - oxo - 3 - quinolinecarboxylic acid trifluoroacetate

1)

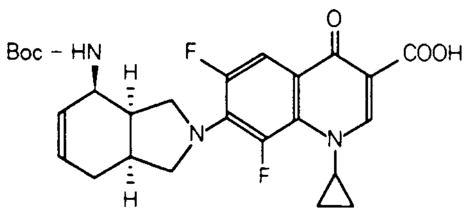

An acetonitrile (5 ml) solution of 1 - cyclopropyl - 6, 7, 8 - trifluoro - 1, 4 - dihydro - 4 - oxo - 3 - quinolinecarboxylic acid ( 113mg, 0.4mmol ) , ( 1R* , 2R* , 6S* ) - 2 - ( N - tert - butoxycarbonylamino) - 8 - azabicyclo [4.3.0] non - 3 - ene (200mg, 0.4mmol) and triethylamine (0.11ml) was refluxed with heating under a nitrogen stream for 4 hours, and then stirred at room temperature for 2 hours. The precipitate was taken by filtration and subjected to silica gel column chromatography (Wakogel™ C-300, 50ml, methylene chloride - methanol 100: 1 → 20 : 1) to obtain 7 - [(1R*, 2R*, 6S*) - 2 - (N - tert - butoxycarbonylamino) - 8 - azabicyclo [4.3.0] non - 3 - en - 8- yl] - 1 - cyclopropyl - 6, 8-difluoro - 1, 4 - dihydro - 4 - oxo - 3 - quinolinecarboxylic acid (70mg, yield : 34.9%).
TLC (Rf) : 0.56 (methylene chloride - methanol 9 : 1)
IR (KBr) $cm^{-1}$ : 3350, 2975, 2940, 2880, 1735, 1690, 1630, 1520, 1450, 1330, 1170, 805
NMR ($CDCl_3$) δ : 1.0~1.7 (4H. m), 1.44 (9H. s), 1.95~2.1 (1H. m), 2.18~2.38 (1H, m), 2.48~2.64 (1H, m), 2.83~3.03 (1H, m), 3.4~5.15 (5H, m), 4.4~4.7 (2H, m), 5.56 (1H, d, J = 10Hz), 5.78~5.9 (1H. m), 7.94 (1H. dd, J = 14, 2Hz), 8.76 (1H, s)
2)

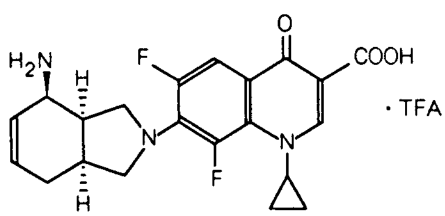

To a methylene chloride (5ml) solution of the compound obtained by the above reaction (62mg, 0.12mmol) and anisole (one drop) was added dropwise, with stirring under ice cooling, a methylene chloride (2ml) solution of trifluoroacetie acid (2ml). The reaction solution was stirred under ice cooling for 2 hours and then the solvent was distilled away under reduced pressure. Diisopropyl ether (15ml) was added to the residue, and the mixture was stirred at room temperature for 2 hours. The precipitate was taken by filtration and recrystallized from ethanol - methylene chloride to obtain the captioned compound (44mg, yield : 69.0%).
TLC (Rf) : 0.63 (ethyl acetate - acetic acid - water 3 : 1 : 1)

MP : 211~212 ° C (decomposed)
IR (KBr) $cm^{-1}$ : 3450, 2950, 1725, 1670, 1630, 1525, 1460, 1360, 1205, 1180, 1145, 805
NMR ($CDCl_3$ - TFA - $d_1$ 1 : 1) δ : 1.2~1.6 (4H, m), 1.9~2.2 (1H, m), 2.4~2.6 (1H, m), 2.7~3.1 (2H, m), 3.7~3.9 (1H, m), 3.9~4.5 (4H, m), 4.5~4.7 (1H, m), 5.76 (1H, d, J = 10Hz), 6.18 (1H, br d, J= 10Hz), 8.02 (1H, dd, J= 14, 2Hz), 9.17 (1H, s)
MS (m/e) : 402 $(M + 1)^+$

Example 2

1 - cyclopropyl - 6, 8 - difluoro - 1, 4 - dihydro - 7 - [(1R*, 2R*, 6S*) - 2 - (N - methylamino) - 8 - azabicyclo [4.3.0] non - 3 - en - 8 - yl] - 4 - oxo - 3 - quinolinecarboxylic acid trifluoroacetate

1)

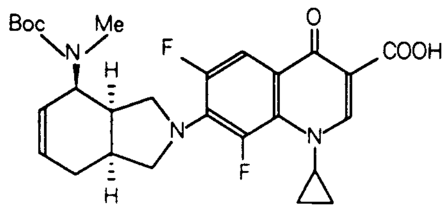

The procedure of Example 1 - 1) was repeated using 1 - cyclopropyl - 6, 7, 8 - trifluoro - 1, 4 - dihydro - 4 - oxo - 3 - quinolinecarboxylic acid (90mg, 0.31mmol),(1R*, 2R*, 6S*) - 2 - (N - tert - butoxycarbonyl - N - methylamino) - 8 - azabicyclo [4.3. 0] non - 3 - ene (80mg, 0.31mmol) and triethylamine (0.09ml) to obtain 7- [(1R*, 2R*, 6S*) - 2 - (N - tert - butoxycarbonyl - N - methylamino) - 8 - azabicyclo [4.3.0] non - 3 - en - 8 - yl] - 1 - cyclopropyl - 6, 8 - difluoro - 1, 4 - dihydro - 4 - oxo - 3 - quinolinecarboxylic acid (107mg, yield : 67%).
TLC (Rf) : 0.71 (methylene chloride - methanol 9 : 1)
IR (KBr) $cm^{-1}$ : 3440, 2980, 2950, 2900, 1735, 1695, 1635, 1530, 1480, 1380, 1370, 1330, 1140, 805
NMR ($CDCl_3$) δ : 1.1~1.7 (4H, m), 1.46 (9H, s), 2.0~2.1 (1H, m), 2.1~2.3 (1H, m), 2.6~3.0 (2H, m), 2.78 (3H, s), 3.3~3.5 (2H, m), 3.8~4.1 (3H m), 4.9~5.1 (1H, m), 5.6 (1H, d, J = 10Hz), 5.8~6.0 (1H, m), 7.84 (1H, dd, J = 14, 2Hz), 8.75 (1H, s)
2)

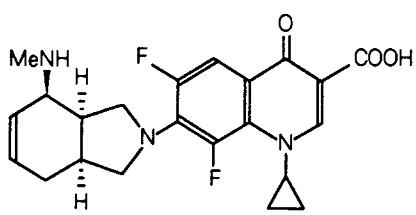

The procedure of Example 1 -2) was repeated using 7-[(1R*, 2R*, 6S*) - 2 - (N - tert - butoxycarbonyl - N - methylamino) - 8 - azabicyclo [4.3.0] non - 3 - en - 8- yl] - 1 - cyclopropyl - 6, 8 - difluoro - 1, 4 - dihydro - 4 - oxo - 3 - quinolinecarboxylic acid (97mg) to obtain the captioned compound (78mg, yield : 78%).
TLC (Rf) : 0.7 (ethyl acetate - acetic acid - water 3 : 1 : 1)
MP : 228 ° C (decomposed)
IR (KBr) $cm^{-1}$ : 3440, 3030, 2900, 2750, 1730, 1690, 1630, 1525, 1465, 1205, 1135, 805
NMR ($CDCl_3$ - TFA - $d_1$ 1 : 1) δ : 1.2~1.7 (4H, m), 2.0~2.2 (1H, m), 2.4~2.7 (1H, m), 2.7~3.2 (2H, m), 3.04 (3H, s), 3.7~3.9 (1H, m), 3.9~4.5 (6H, m), 5.92 (1H, d, J = 10Hz), 6.2~6.3 (1H, m), 8.0 (1H, dd, J = 14, 2Hz), 9.16 (1H, s)
MS (m/e) : 416 $(M + 1)^+$

Example 3

7 - [(1R*, 2R*, 6S*) - 2 - amino - 8 - azabicyclo [4.3.0] non - 3 - en - 8 - yl] - 1 - cyclopropyl - 6, 8 - difluoro - 1, 4 - dihydro - 4 - oxo - 3 - quinolinecarboxylic acid hydrochloride

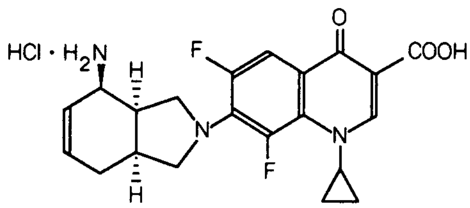

7 - [(1R*, 2R*, 6S*) - 2 - (N - tert - butoxycarbonylamino) - 8 - azabicyclo [4.3.0] non - 3 - en - 8 - yl] - 1 - cyclopropyl - 6, 8 - difluoro - 1, 4 - dihydro - 4 - oxo - 3 - quinolinecarboxylic acid (302mg, 0.6mmol) obtained in Example 1 - 1) was dissolved in a 2N hydrochloric acid methanol solution (5ml). The resultant solution was stirred at room temperature for 30 minutes, the solvent was distilled away under reduced pressure, and the residue was recrystallized from ethanol to obtain the captioned compound (247mg, yield : 87.7%).

TLC (Rf) : 0.63 (ethyl acetate - acetic acid - water 3 : 1 : 1)

MP : 238 °C (decomposed)

IR (KBr) $cm^{-1}$ : 3450, 2880, 1735, 1630, 1520, 1460, 1410, 1350, 1325, 805

NMR ($CDCl_3$ - TFA - $d_1$ 1 : 1) δ : 1.2~1.6 (4H, m), 2.0~2.2 (1H, m), 2.4~2.6 (1H. m), 2.74~2.92 (1H, m), 2.97~3.15 (1H, m), 3.75~3.87 (1H, m), 4.0~4.5 (4H, m), 4.57~4.7 (1H, m), 5.67 (1H, br d, J = 10Hz), 6.18 (1H, br d, J = 10Hz), 8.0 (1H, dd, J = 2, 14Hz), 9.15 (1H, s)

MS (m/e) : 402 (M + 1)

Example 4

7 - [(1R*, 2R*, 6S*) - 2 - amino - 8 - azabicyclo [4.3.0] non - 3 - en - 8 - yl] - 1 - cyclopropyl - 6 - fluoro - 1, 4 - dihydro - 4 - oxo - 3 - quinolinecarboxylic acid hydrochloride

1)

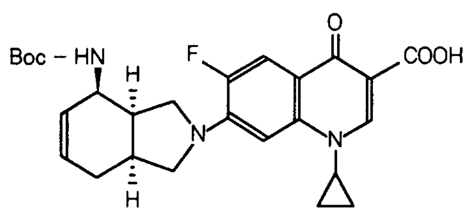

An acetonitrile (5ml) solution of 1 - cyclopropyl - 6, 7 - difluoro - 1, 4 - dihydro - 4 - oxo - 3 - quinolinecaboxylic acid (133mg, 0.5mmol ; prepared according to Japanese Published Unexamined Patent Application No.126271/1985), (1R*, 2R*, 6S*) - 2 - (N - tert - butoxycarbonylamino) - 8 - azabicyclo [4.3.0] non - 3 - ene (119mg, 0.5mmol) and triethylamine (0.14ml) was refluxed with heating for 4 hours, with stirring under a nitrogen stream, and then stirred at room temperature for 16 hours. The precipitate was taken by filtration and washed with acetonitrile to obtain 7 - [ ( 1R* , 2R* , 6S* ) - 2 - ( N - tert - butoxycarbonylamino) - 8 - azabicyclo [4.3.0] non - 3 - en - 8 - yl] - 1 - cyclopropyl - 6 - fluoro - 1, 4 - dihydro - 4 - oxo - 3 - quinolinecarboxylic acid (188mg, yield : 77.8%).

IR (KBr) $cm^{-1}$ : 3440, 3340, 2970, 1715, 1635, 1510, 1470, 1360, 820

NMR ($CDCl_3$) δ : 1.1~1.8 (4H, m), 1.44 (9H, s), 1.9~2.1 (1H, m), 2.2~2.43 (1H, m), 2.55~2.7 (1H, m), 2.9~3.1 (1H, m), 3.3~ 3.8 (4H, m), 4.5~4.7 (2H, m), 5.5~5.63 (1H, m), 5.8~5.92 (1H, m), 6.87 (1H, d, J = 8Hz), 7.92 (1H, d, J = 14Hz), 8.67 (1H, s)

2)

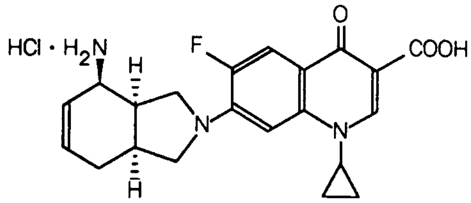

The procedure of Example 3 was repeated using a 2N hydrochloric acid methanol solution (5ml) of the compound obtained by the above reaction (180mg, 0.37mmol) to obtain the captioned compound (145mg, yield : 92.8%).

TLC (Rf) : 0.61 (ethyl aeetate - acetic acid - water 3 : 1 : 1)

MP : 250 ° C or more

IR (KBr) $cm^{-1}$ : 3430, 3220, 2890, 2830, 2600, 1725, 1640, 1515, 1480, 1465, 1395, 1360

NMR ($CDCl_3$ - TFA - $d_1$ 1 : 1) δ : 1.2~1.4 (2H, m), 1.5~1.7 (2H, m), 1.9~2.1 (1H, m), 2.2~2.7 (1H, m), 2.8~3.0 (1H, m), 3.0~3.3 (1H, m), 3.7~4.2 (5H, m), 4.6~4.7 (1H, m), 5.7~5.9 (1H, m), 6.1~6.3 (1H, m), 7.3 (1H, br d, J = 8Hz), 8.1 (1H, d, J = 14Hz), 9.1 (1H, s)

MS (m/e) : 384 $(M + 1)^+$

Example 5

7 - [1R*, 2R*, 6S*) - 2 - amino - 8 - azabicyclo [4.3.0] non - 3 - en - 8 - yl] - 6, 8 - difluoro - 1 - (2, 4 - difluorophenyl) - 1, 4 - dihydro - 4 - oxo - 3 - quinolinecarboxylic acid hydrochloride

1)

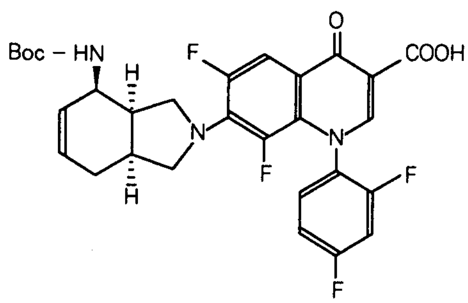

The procedure of Example 4 - 1) was repeated using an acetonitrile (5ml) solution of 6, 7, 8 - trifluoro - 1 - (2, 4 - difluorophenyl) - 1, 4 - dihydro - 4 - oxo - 3 - quinolinecarboxylic acid [178mg, 0.5mmol ; prepared according to J. Med. Chem. 28, 1558 (1985 ) ] , ( 1R* , 2R* , 6S* ) - 2 - ( N - tert - butoxycarbonylamino) - 8 - azabicyclo [4.3.0] non - 3 - ene (119mg, 0.5mmol) and triethylamine (0.14ml) to obtain 7 - [(1R*, 2R*, 6S*) - 2 - (N - tert - butoxycarbonylamino) - 8 - azabicyclo [4.3. 0] non - 3 - en - 8 - yl] - 6, 8 - difluoro - 1 - (2, 4 - difluorophenyl) - 1, 4 - dihydro - 4 - oxo - 3 - quinolinecarboxylic acid (225mg, yield : 78.5%).

IR (KBr) $cm^{-1}$ : 3420, 3360, 1735, 1690, 1630, 1515, 1500, 1465, 1410, 1340, 805

NMR ($CDCl_3$ - $CD_3OD$ 6 : 1) δ : 1.44 (9H, s), 1.8~2.0 (1H, m), 2.1~ 2.3 (1H, m), 2.4~2.6 (1H, m), 2.7~2.9 (1H, m), 3.3~4.0 (4H, m). 4.4~4.6 (1H, m), 5.0~5.2 (1H, m), 5.4~5.55 (1H, m), 5.7~5.86 (1H, m), 7.0~7.2 (2H, m), 7.4~7.6 (1H, m), 7.9 (1H, dd, J = 14, 2Hz), 8.47 (1H, d, J = 2Hz)

2)

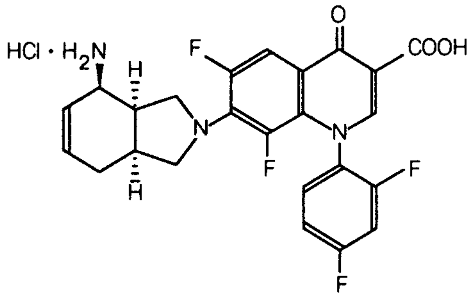

The procedure of Example 3 was repeated using a 2N hydrochloric acid methanol solution (5ml) of the compound obtained by the above reaction (210mg, 0.37mmol) to obtain the captioned compound (147mg, yield : 78.8%).
TLC (Rf) : 0.65 (ethyl acetate - acetic acid - water 3 : 1 : 1)
MP : 220 ° C (decomposed)
IR (KBr) $cm^{-1}$ : 3420, 2950, 2890, 1725, 1630, 1515, 1465, 1410, 1355, 1145, 970, 805, 540
NMR ($CDCl_3$ - TFA - $d_1$ 1 : 1) δ : 1.9~2.1 (1H, m), 2.4~2.6 (1H, m), 2.6~2.9 (1H, m), 2.9~3.1 (1H, m), 3.6~3.8 (1H, m), 3.9~4.3 (3H, m), 4.5~4.65 (5H, m), 5.65~5.8 (1H, m), 6.05~6.2 (1H, m), 7.1~7.3 (2H, m), 7.6~7.7 (1H, m), 8.1 (1H, d, J = 14Hz), 8.88 (1H, s)
MS (m/e) : 474 $(M + 1)^+$

Example 6

7 - [(1R* 2R*, 6S*) - 2 - amino - 8 - azabicyclo [4.3.0] non - 3 - en - 8 - yl] - 1 - ethyl - 6 - fluoro - 1, 4 - dihydro - 4 - oxo - 3 - quinolinecarboxylic acid

1)

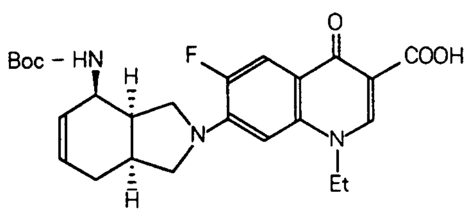

The procedure of Example 4 - 1) was repeated using an acetonitrile (5ml) solution of 1 - ethyl - 6, 7 - difluoro - 1, 4 - dihydro- 4 - oxo - 3 - quinolinecarboxylic acid (132mg, 0.5mmol ; prepared according to Japanese Published Unexamined Patent Application No.30964/1981), (1R*, 2R*, 6S*) - 2 - (N - tert - butoxycarbonylamino) - 8 - azabicyclo [4.3.0] non - 3 - ene (119mg, 0.5mmol) and triethylamine (0.14ml) to obtain 7 - [(1R*, 2R*, 6S*) - 2 - (N - tert - butoxycarbonylamino) - 8 - azabicyclo [4.3. 0] non - 3 - en - 8 - yl] - 1 - ethyl - 6 - fluoro - 1, 4 -dihydro-4 - oxo - 3 - quinolinecarboxylic acid (179mg, yield : 74.4%).
IR (KBr) $cm^{-1}$ : 3420, 3350, 2980, 1740, 1725, 1690, 1635, 1520, 1470, 1370, 1170, 815
NMR ($CDCl_3$) δ : 1.44 (9H, s), 1.57 (3H t, J = 7Hz), 1.8~2.05 (1H, m), 2.2~2.42 (1H, m), 2.58~2.7 (1H, m), 2.9~3.1 (1H, m), 3.36~3.8 (4H, m), 4.28 (2H, q, J = 7Hz), 4.5~4.7 (2H, m), 5.5~5.6 (1H, m), 5.78~5.9 (1H, m), 6.36 (1H, d, J = 8Hz), 8.0 (1H, d, J = 14Hz), 8.6 (1H, s)
2)

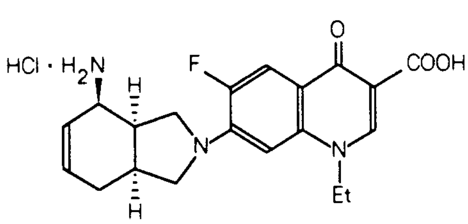

The procedure of Example 3 was repeated using a 2N hydrochloric acid methanol solution (5ml) of the compound obtained by the above reaction (174mg, 0.36mmol) to obtain the captioned compound (145mg, yield : 95.8%).
TLC (Rf) : 0.55 (ethyl acetate - acetic acid - water 3 : 1 : 1)
MP : 250 °C or more
IR (KBr) $cm^{-1}$ : 3420, 3220, 2890, 1705, 1635, 1520, 1475, 1465, 1410, 1390, 1355
NMR ($CDCl_3$ - TFA - $d_1$ 1 : 1) δ : 1.6~1.8 (3H, m), 1.9~2.1 (1H, m), 2.4~2.6 (1H, m), 2.8~3.0 (1H, m), 3.0~3.25 (1H, m), 3.5~4.2 (5H, m), 4.55~4.8 (2H, m), 5.7~5.85 (1H, m), 6.05~6.2 (1H, m), 6.8~7.0 (1H, m), 8.1 (1H, d, J = 14Hz), 9.05 (1H, s)

Example 7

7 - [(1R*, 2R*, 6S*) - 2 - amino - 8 - azabicyclo [4.3.0] non - 3 - en - 8 - yl] - 1 - cyclopropyl - 6 - fluoro - 1, 4 - dihydro - 8 - methoxy - 4 - oxo - 3 - quinolinecarboxylic acid hydrochloride

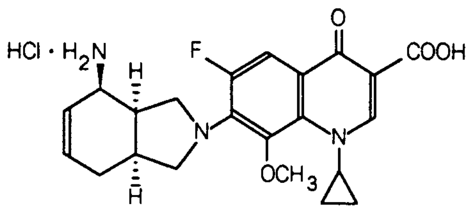

An acetonitrile (30ml) solution of 1 - cyclopropyl - 6, 7 - difluoro - 1 , 4 - dihydro - 8 - methoxy - 4 - oxo - 3 - quinolinecaboxylic acid (1.77g, 6mmol ; prepared according to Japanese Published Unexamined Patent Application No.198664/1988),(1R*, 2R*, 6S*) - 2 - amino - 8 - azabicyclo [4.3.0] non - 3 - ene dihydrochloride (1.27g, 6mmol) and 1, 8 - diazabicyclo [5.4.0] undec - 7 - ene (2.96ml) was refluxed with heating for 5 hours, with stirring under a nitrogen stream, and then stirred at room temperature for 13 hours. The precipitate was taken by filtration and dissolved in a 2N hydrochloric acid methanol solution (20ml), and then the solvent was distilled away under reduced pressure. The resultant residue was dissolved in 80 ml of methylene chloride - ethanol (2 : 1), methylene chloride was distilled away under reduced pressure, and the resultant mixture was allowed to stand at room temperature for 2 hours. The precipitated crystals were taken by filtration and washed with ethanol to obtain the captioned compound (1.35g, yield : 50.0%).
TLC (Rf) : 0.64 (ethyl acetate - acetic acid - water 3 : 1 : 1)
MP : 205 °C (decomposed)
IR (KBr) $cm^{-1}$ : 3450, 2940, 2880, 1710, 1620, 1510, 1450, 1350, 1320, 1060, 800
NMR ($CDCl_3$ - TFA - $d_1$ 1 : 1) δ : 0.9~1.1 (1H, m), 1.2~1.5 (2H, m), 1.5~1.7 (1H, m), 1.9~2.2 (1H, m), 2.4~2.7 (1H, m), 2.8~3.0 (1H, m), 3.0~3.2 (1H, m), 3.6~3.8 (1H, m), 3.66 (3H, s), 3.8~4.0 (1H, m), 4.0~4.7 (4H, m), 5.7~5.85 (1H, m), 6.1~6.3 (1H, m), 7.98 (1H, d, J = 14Hz), 9.2 (1H, s)
MS (m/e) : 414 $(M + 1)^+$

Example 8

7 - [(1R*, 2R*, 6S*) - 2 - amino - 8 - azabicyclo [4.3.0] non - 3 - en - 8 - yl] - 1 - cyclopropyl - 6, 8 - difluoro - 1, 4 - dihydro - 4 - oxo - 3 - quinolinecarboxylic acid hydrochloride

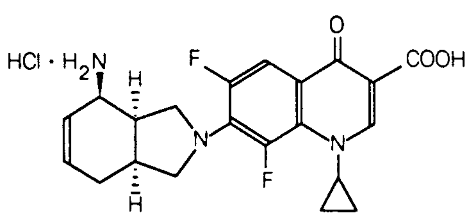

The procedure of Example 7 was repeated using an aeetonitrile (15ml) solution of 1 - cyclopropyl - 6, 7, 8 - trifluoro - 1, 4 - dihydro - 4 - oxo - 3 - quinolinecarboxylic acid (849mg, 3mmol),(1R*, 2R*, 6S*) - 2 - amino - 8 - azabicyclo [4.3.0] non - 3-ene dihydrochloride (633mg, 3mmol) and 1, 8 - diazabicyclo [5. 4.0] undec - 7 - ene (1.48ml, 9.8mmol) to obtain the captioned compound (700mg, yield : 60.2%). The instrumental analysis data coincided with the data of the compound of Example 3.

Example 9

(-) - 7 - [(1R*, 2R*, 6S*) - 2 - amino - 8 - azabicyclo [4.3.0] non - 3 - en - 8 - yl] - 1 - cyclopropyl - 6, 8 - difluoro - 1, 4 - dihydro - 4 - oxo - 3 - quinolinecarboxylic acid hydrochloride

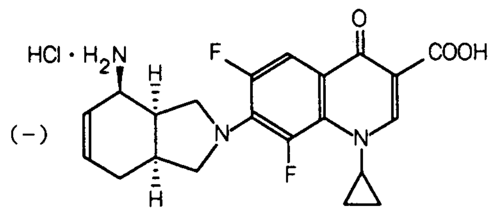

The procedure of Example 7 was repeated using an aeetonitrile (5ml) solution of 1 - cyclopropyl - 6, 7, 8 - trifluoro - 1, 4 - dihydro - 4 - oxo - 3 - quinolinecarboxylic acid (283mg, 1mmol),(1R*, 2R*, 6S*) - 2 - amino - 8 - azabicyclo [4.3.0] non - 3 - ene L - (+) - tartrate (288mg, 1mmol) and 1, 8 - diazabicyclo [5.4.0] undec - 7 - ene (0.448ml) to obtain the captioned compound (260mg, yield : 59.4%).
$[\alpha]_D^{20}$ : - 192.5° (C = 0.5, 0.01N HCl)
TLC (Rf) : 0.60 (ethyl acetate - acetic acid - water 3 : 1 : 1)
MP : 201 °C (decomposed)
IR (KBr) $cm^{-1}$ : 3450, 2900, 1720, 1625, 1520, 1460, 1350, 805
NMR ($CDCl_3$ -TFA - $d_1$ 1 : 1) δ : 1.2~1.6 (4H, m), 1.96~2.2 (1H, m), 2.4~2.6 (1H, m), 2.74~2.94 (1H, m), 2.96~3.16 (1H, m), 3.75~3.85 (1H, m), 4.0~4.5 (4H, m), 4.58~4.7 (1H, m), 5.76 (1H, br d. J = 10Hz), 6.18 (1H, br d, J = 10Hz), 7.98 (1H, dd, J = 14, 2Hz), 9.14 (1H, s)

Example 10

(+) - 7 - [(1R*, 2R*, 6S*) - 2 - amino - 8 - azabicyclo [4.3.0] non - 3 - en - 8 - yl] - 1 - cyclopropyl - 6, 8 - difluoro- 1, 4 - dihydro - 4 - oxo - 3 - quinolinecarboxylic acid hydrochloride

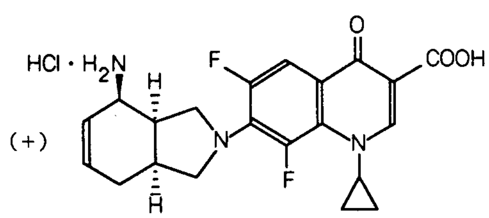

The procedure of Example 7 was repeated using an acetonitrile (5ml) solution of 1 - cyclopropyl - 6, 7, 8 - trifluoro - 1, 4 - dihydro - 4 - oxo - 3 - quinolinecarboxylic acid (283mg, 1mmol),(1R*, 2R*, 6S*) - 2 - amino - 8 - azabicyclo [4.3.0] non - 3 - ene D - (-) - tartrate (288mg, 1mmol) and 1, 8 - diazabicyclo [5.4.0] undec - 7 - ene (0.448ml) to obtain the captioned compound (240mg, yield : 54.8%).
$[\alpha]_D^{20}$ : + 202.7° (C = 0.5, 0.01N HCl)
TLC (Rf) : 0.61 (ethyl acetate - acetic acid - water 3 : 1 : 1)
MP : 201 °C (decomposed)
IR (KBr) $cm^{-1}$ : 3450, 2900, 1720, 1625, 1520, 1460, 1350, 805
NMR ($CDCl_3$ - TFA - $d_1$ 1 : 1) δ : 1.2~1.6 (4H, m), 1.95~2.2 (1H, m), 2.4~2.62 (1H, m), 2.74~2.9 (4H, m), 2.95~3.14 (1H, m), 3.74~3.9 (1H, m), 4.0~4.5 (4H, m), 4.58~4.68 (1H, m), 5.76 (1H, br d, J = 10Hz), 6.17 (1H, br d, J = 10Hz), 7.98 (1H, dd, J= 14, 2Hz), 9.14 (1H, s)

Example 11

(-) - 7 - [(1R*, 2R*, 6S*) - 2 - amino - 8 - azabicyclo [4.3.0] non - 3 - en - 8 - yl] - 1 - cyclopropyl - 6 - fluoro - 1, 4 - dihydro - 8 - methoxy - 4 - oxo - 3 - quinolinecarboxlic acid hydrochloride

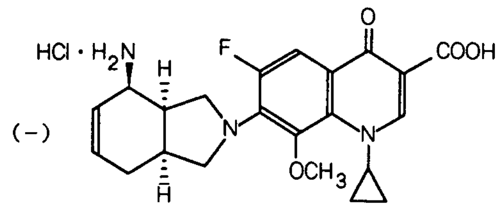

An acetonitrile (15ml) solution of 1 - cyclopropyl - 6, 7 - difluoro - 1 , 4 - dihydro - 8 - methoxy - 4 - oxo - 3 - quinolinecaboxylic acid (443mg, 1.5mmol),(1R*, 2R*, 6S*) - 2 - amino - 8 - azabicyclo [4.3.0] non - 3 - ene L - (+) - tartrate (432mg, 1.5mmol) and 1, 8 - diazabicyclo [5.4.0] undec - 7 - ene (0. 672ml) was refluxed with heating for 4 hours, with stirring under a nitrogen stream. The solvent of the reaction solution was distilled away under reduced pressure, a 2N hydrochloric acid methanol solution (5ml) was added to dissolve the residue, and then the solvent was distilled away under reduced pressure. The residue was subjected to silica gel column chromatography (Wakogel™ C - 300, 40ml, methylene chloride - methanol 100 : 1 → 20 : 1) and then recrystallized from ethanol to obtain the captioned compound (64mg, yield : 9.5%).

$[\alpha]_D^{20}$ : - 166.5° (C = 0.5, 0.01N HCl)

TLC (Rf) : 0.61 (ethyl acetate - acetic acid - water 3 : 1 : 1)

MP : 182 °C (decomposed)

IR (KBr) $cm^{-1}$ : 3440, 2940, 2880, 1710, 1620, 1515, 1450, 1350, 1060, 800

NMR ($CDCl_3$ - TFA - $d_1$ 1 : 1) $\delta$ : 0.9~1.1 (1H, m), 1.22~1.42 (2H, m), 1.48~1.68 (1H, m), 1.92~2.18 (1H, m), 2.42~2.64 (1H, m), 2.78~2.96 (1H, m), 2.98~3.18 (1H, m), 3.58~3.96 (2H, m), 3.66 (3H, s), 4.1~4.5 (3H, m), 4.58~4.7 (1H, m), 5.76 (1H, br d, J = 10Hz), 6.1~6.25 (1H, m), 7.96 (1H, d, J = 14Hz), 9.18 (1H, s)

Example 12

(+) - 7 - [(1R*, 2R*, 6S*) - 2 - amino - 8 - azabicyclo [4.3.0] non - 3 - en - 8 - yl] - 1 - cyclopropyl - 6 - fluoro - 1, 4 - dihydro - 8 - methoxy - 4 - oxo - 3 - quinolinecarboxylic acid hydrochloride

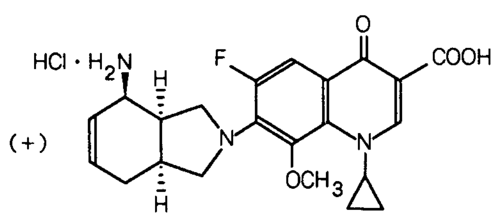

The procedure of Example 11 was repeated using an acetonitrile (5ml) solution of 1 - cyclopropyl - 6, 7 - difluoro - 1, 4 - dihydro - 8 - methoxy - 4 - oxo - 3 - quinolinecarboxylic acid (443mg, 1.5mmol),(1R*, 2R*, 6S*) - 2 - amino - 8 - azabicyclo [4. 3.0] non - 3 - ene D - (-) - tartrate (432mg, 1.5mmol) and 1, 8 - diazabicyclo [5.4.0] undec - 7 - ene (0.672ml) to obtain the captioned compound (90mg, yield : 13.3%).

$[\alpha]_D^{20}$ : + 186.7 ° (C = 0.5, 0.01N HCl)

TLC (Rf) : 0.61 (ethyl acetate - acetic acid - water 3 : 1 : 1)

MP : 182 °C (decomposed)

IR (KBr) $cm^{-1}$ : 3440, 2940, 2880, 1710, 1620, 1515, 1450, 1350, 1060, 800

NMR ($CDCl_3$ - TFA - $d_1$ 1 : 1) $\delta$ : 0.9~1.1 (1H, m), 1.2~1.45 (2H, m), 1.5~1.68 (1H, m), 1.92~2.14 (1H, m), 2.4~2.6 (1H, m), 2.77~2.93 (1H, m), 2.97~3.17 (1H, m), 3.5~3.7 (1H, m), 3.65 (3H, s), 3.7~3.95 (1H, m), 4.08~4.5 (3H, m) 4.58~4.68 (1H, m), 5.76 (1H, br d, J = 10Hz), 6.1~6.24 (1H, m), 7.96 (1H, d, J = 14Hz), 9.18 (1H, s)

Example 13

(-) - 5 - amino - 7 - [(1R*, 2R*, 6S*) 2 - amino - 8 - azabicyclo [4.3.0] non - 3 - en - 8 - yl] - 1 - cyclopropyl - 6, 8 - difluoro - 1, 4 - dihydro - 4 - oxo - 3 - quinolinecarboxylic acid hydrochloride

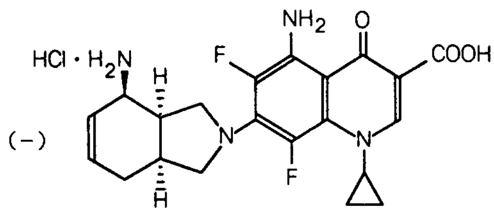

The procedure of Example 7 was repeated using an acetonitrile (5ml) solution of 5 - amino - 1 - cyclopropyl - 6, 7, 8 - trifluoro - 1, 4 - dihydro - 4 - oxo - 3 - quinolinecarboxylic acid [298mg, 1mmol ; prepared according to J. Med. Chem. 33, 1645 (1990)], (+) - (1R*, 2R*, 6S*) - 2 - amino - 8- azabicyclo [4.3.0] non - 3 - ene dihydrochloride (211mg, 1mmol) and 1, 8 - diazabicyclo [5.4.0] undec - 7 - ene (0.45ml, 3mmol) to obtain the captioned compound (390mg, yield : 86.2%).

$[\alpha]_D^{20}$ : - 166.5 ° (C = 0.05, 0.1N hydrochloric acid methanol - 0.1N hydrochloric acid - methylene chloride 3 : 1 : 1)

TLC (Rf) : 0.41 (methylene chloride - methanol - acetic acid 45 : 5 : 1)

MP : 250 °C or more

IR (KBr) $cm^{-1}$ : 3410, 3300, 2940, 2880, 2820, 1690, 1620, 1590, 1515, 1430, 1350, 1305

NMR ($CDCl_3$ - TFA - $d_1$ 1 : 1) δ : 1.1~1.52 (4H, m), 1.95~2.2 (1H, m), 2.4~2.6 (1H, m), 2, 72~2.90 (1H, m), 2.92~3.14 (1H, m), 3.6~3.78 (1H, m), 3.9~4.36 (4H, m), 4.56~4.7 (1H, m), 5.75 (1H, br s, J = 9Hz), 6.1~6.25 (1H, m), 9.01 (1H, s)

MS (m/e) : 417 $(M + 1)^+$

Example 14

(+) - 5 - amino - 7 - [(1R*, 2R*, 6S*) - 2 - amino - 8 - azabicyclo [4.3.0] non - 3 - en - 8 - yl] - 1 - cyclopropyl - 6, 7 - difluoro - 1, 4 - dihydro - 4 - oxo - 3 - quinolinecarboxylic acid hydrochloride

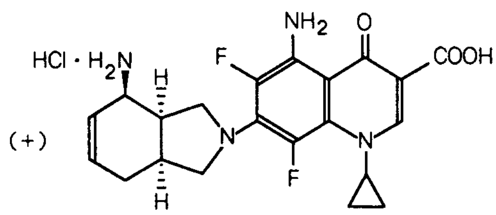

The procedure of Example 7 was repeated using 5 - amino - 1 - cyclopropyl - 6, 7, 8 - trifluoro - 1, 4 - dihydro - 4 - oxo - 3 - quinolinecarboxylic acid (298mg, 1mmol), (-) - (1R*, 2R*, 6S*) - 2 - amino - 8 - azabicyclo [4.3.0] non - 3 - ene dihydrochloride (211mg, 1mmol) and triethylamine (0.56ml, 4mmol) to obtain the captioned compound (80mg, yield : 17.7%).

$[\alpha]_D^{20}$ : + 158.2 ° (C = 0.05, 0.1N hydrochloric acid methanol - 0.1N hydrochloric acid - methylene chloride 3 : 1 : 1)

TLC (Rf) : 0.41 (methylene chloride - methanol - acetic acid 45 : 5 : 1)

MP : 250 °C or more

IR (KBr) $cm^{-1}$ : 3410, 3300, 2940, 2820, 1690, 1620, 1590, 1515, 1430, 1350, 1315

NMR ($CDCl_3$ - TFA - $d_1$ 1 : 1) δ : 1.1~1.52 (4H, m), 1.95~2.2 (1H, m), 2.4~2.6 (1H, m), 2, 72~2.90 (1H, m), 2.92~3.14 (1H, m), 3.6~3.78 (1H, m), 3.9~4.36 (4H, m), 4.56~4.7 (1H, m), 5.75 (1H, br s, J = 9Hz), 6.1~6.25 (1H, m), 9.01 (1H, s)

MS (m/e) : 417 $(M + 1)^+$

Example 15

98 parts of lactose is added to 1 part of the compound of Example 1 to prepare a 100 - fold trituration. To this powder is added a kneading liquid prepared by adding purified water to 1 part of hydroxypropylcellulose, and the mixture is kneaded and granulated. The granules are passed through a 20 mesh sieve for classification, dried, and sieved out into a specific grain size to give granules.

Example 16

20 parts of lactose and 5 parts of corn starch are added to 1 part of the compound of Example 1, and the mixture is sufficiently mixed. By conventional methods, the resultant mixture is granulated with ethanol, dried and milled. 0.5 % magnesium stearate is added thereto, and, after mixing, the mixture is made into tablets each containing 10 mg of the compound of Example 1, according to a conventional method.

Example 17

1 weight part of the compound of Example 1, 22 weight parts of lactose and 6.8 weight parts of corn starch are mixed, and 3.4 weight parts of a corn starch glue liquid (prepared by adding 30 ml of water to 4.0 weight parts of corn starch and stirring the mixture under heating) is added thereto. The mixture is kneaded, granulated by an extrusion granulator and dried at 50 °C under reduced pressure for 4 hours. The resultant dried granulates are milled by a Fitzmill® to obtain 32 weight parts of granules having a particle size of 20 to 35 meshes. 32 weight parts of the granules are put in a coating pan and subjected to conventional spray coating using an alcohol solution of Eudragit L® to obtain 33 weight parts of enteric granules containing the compound of Example 1.

Reference example 1

(1R*, 2R*, 6S*) - 2 - (N - tert - butoxycarbonylamino) - 8 - azabicyclo [4.3.0] non - 3 - ene

1)

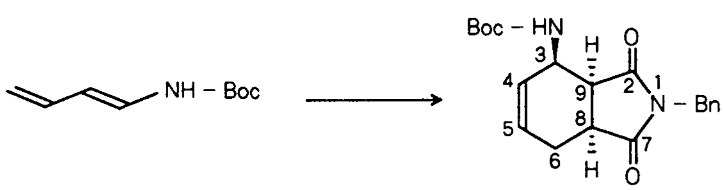

A dioxane (10ml) solution of (E) - 1 - (N - tert - butoxycarbonylamino) - 1, 3 - butadiene [4.23g, 25mmol, prepared according to Organic Syntheses 6, 95 (1988) ] , N - benzylmaleinimide (4.68g, 25mmol) and 4 - tert - butylcatechol (50mg) was stirred at 80 to 90 °C for 1 hour. The solvent was distilled away under reduced pressure, and the residue was subjected to silica gel column chromatography (Wakogel™ C - 300, 200ml, hexane - ethyl acetate 10 : 1 → 1 : 1) to obtain (3R*, 8S*, 9S*) - 3 - (N - tert - butoxycarbonylamino) - 1 - benzyl - 3, 6, 8, 9 - tetrahydrophthalimide (6.09g, yield : 68.4%).
TLC (Rf) : 0.5 (hexane - ethyl acetate 2 : 1)
IR (KBr) $cm^{-1}$ : 3340, 2990, 1780, 1710, 1680, 1540, 1400, 1360, 1170
NMR ($CDCl_3$) δ : 1.46 (9H, s), 2.20 (1H, dd, J = 8, 16Hz), 2.68 (1H, dd, J = 7, 16Hz), 3.1~3.3 (2H, m), 4.3~4.5 (1H, m), 4.62 (2H, s), 5.80 (2H, br s), 6.27 (1H, br s), 7.29 (5H, s)
2)

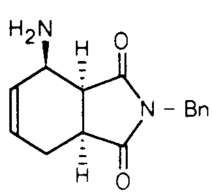

To a methylene chloride (20ml) solution of the compound obtained by the above reaction (6.09g, 17mmol) and anisole (3 drops) was added dropwise, under ice cooling and stirring, a methylene chloride (6ml) solution of trifluoroacetic acid (6ml). The reaction solution was stirred under ice cooling for 2 hours and then at room temperature for 3 hours. The solvent was distilled away under reduced pressure, diisopropyl ether (30ml) was added, and the mixture was stirred overnight. The precipitate was taken by filtration and dissolved in water, and ethyl acetate was added. The mixture was neutralized, with stirring, with a 5% aqueous sodium bicarbonate solution. The organic layer was dried over anhydrous sodium sulfate and the solvent was distilled away under reduced pressure to obtain (3R*, 8S* , 9S* ) - 3 - amino - 1 - benzyl - 3 , 6 , 8 , 9 - tetrahydrophthalimide (3.2g, yield : 73.5%).
TLC (Rf) : 0.32 (methylene chloride - methanol 9 : 1)
IR (KBr) $cm^{-1}$ : 3380, 3040, 1770, 1700, 1430, 1405, 1360, 1195
NMR ($CDCl_3$) δ : 2.1~2.3 (1H, m), 2.68 (1H, dd, J = 8, 16Hz), 3.1~ 3.2 (2H, m), 3.66 (1H, br s), 4.63 (2H, s), 5.8~6.0 (2H, m), 7.2~7.5 (5H, m)
3)

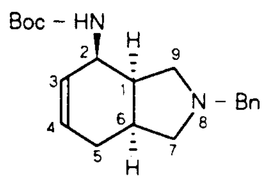

A tetrahydrofuran (30ml) solution of the compound obtained by the above reaction (3.07g, 12mmol) was added dropise to a tetrahydrofuran (20ml) solution of lithium aluminum hydride (1.4g, 36mmol) at 0 ° C with stirring. The reaction solution was refluxed with heating for 4 hours, and ethyl acetate (10ml) and then 3N hydrochloric acid (30ml) were gradually added dropwise to the reaction mixture under ice cooling. The aqueous layer was separated, made alkaline with a 20% aqueous sodium hydroxide solution, and extracted three times with ethyl actate. The organic layer was dried over anhydrous sodium sulfate and the solvent was distilled away under reduced pressure to obtain crude (1R*, 2R*, 6S*) - 2 - amino - 8 - azabicyclo [4.3.0] non - 3 - ene (3.0g) as the residue. Boc - S (2.2g, 9.2mmol) and triethylamine (1.4ml, 10mmol) were added to a dioxane (30ml) solution of this residue, and the resultant solution was stirred at room temperature overnight. After removal of the precipitate by filtration, the solvent in the filtrate was distilled away under reduced pressure and the residue was dissolved in ethyl acetate. This solution was washed three times with a 5% aqueous sodium bicarbonate solution and dried over anhydrous sodium sulfate, and the solvent was distilled away under reduced pressure. Diethyl ether was added to the residue, and the precipitate was taken by filtration which was (1R*, 2R*, 6S*) - 8 - benzyl - 2 - (N - tert - butoxycarbonylamino) - 8 - azabicyclo [4.3.0] non - 3 - ene (1.15g, yield : 29.2%).
TLC (Rf) : 0.22 (hexane - ethyl acetate 1 : 1)
IR (KBr) $cm^{-1}$ : 3375, 2945, 2800, 1680, 1520, 1255, 1170
NMR ($CDCl_3$) δ : 1.44 (9H, s), 1.8~2.9 (8H, m), 3.61 (2H, s), 4.2~ 4.3 (1H, m), 5.33 (1H, br), 5.7~5.95 (2H, m), 7.2~7.5 (5H, m)
4)

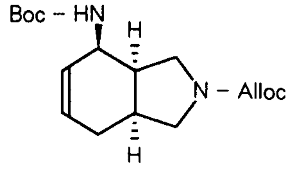

To a 1, 2 - dichloroethane (10ml) solution of the compound obtained by the above reaction (612mg, 1.86mmol) was added, under ice cooling and stirring, allyl chloroformate (0. 395ml). The reaction solution was stirred at 0 ° C for 20 minutes and then refluxed with heating for 1 hour. The reaction solution was cooled, the solvent was distilled away under reduced pressure, and the residue was purified by silica gel column chromatography (Wakogel™ C - 300, 70ml, hexane - ethyl acetate 10 : 1 → 1 : 1) to obtain (1R*, 2R*, 6S*) - 8 - allyloxycarbonyl - 2 - (N - tert - butoxycarbonylamino) - 8 - azabicyclo [4.3.0] non - 3 - ene (439mg, yield : 73.1%).
TLC (Rf) : 0.56 (hexane - ethyl acetate 1 : 1)

IR (KBr) $cm^{-1}$ : 3360, 1720, 1685, 1520, 1430, 1170
NMR ($CDCl_3$) δ : 1.44 (9H, s), 1.8~2.0 (1H, m), 2.1~2.5 (2H, m), 2.7~2.95 (1H, m), 3.17 (1H, t, J = 10Hz), 3.3~3.6 (3H, m), 4.4~4.65 (4H, m), 5.2~5.35 (3H, m), 5.7~6.1 (2H, m)
5)

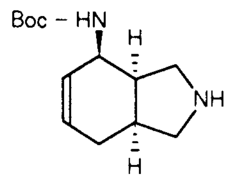

A methylene chloride (30ml) solution of the compound obtained by the above reaction (439mg, 1.36mmol), acetic acid (0. 195ml, 3.4mmol) and bis (triphenylphosphine) palladium ( II ) chloride (19.1mg) was deaerated. While the solution was intensely stirred at 0°C under a nitrogen stream, tributyltin hydride (0. 727ml, 2.72mmol) was added all at once. The mixture was stirred at 0°C for 5 minutes and then at room temperature for 20 minutes, and thereafter the solvent was distilled away under reduced pressure. Ethyl acetate (20ml) and water (20ml) were added to the residue, and the mixture was adjusted to pH 7.5. The aqueous layer was separated, and subjected to reverse - phase column chromatography (YMC - GEL™ ODS - AQ 120 - S50, 50ml, methanol ) to obtain ( 1R* , 2R* , 6S* ) - 2 - ( N - tert - butoxycarbonylamino) - 8 - azabicyclo [4.3.0] non - 3 - ene (119mg, yield : 36.8%).
TLC (Rf) : 0.08 (methylene chloride - methanol 9 : 1)
IR (KBr) $cm^{-1}$ : 3350, 2970, 2930, 1705, 1690, 1575, 1525, 1390, 1360, 1245, 1170
NMR ($CDCl_3$ - $CD_3OD$ 5 : 1) δ : 1.46 (9H, m), 1.82~2.0 (1H, m), 2.1~2.3 (1H, m), 2.4~2.6 (1H, m), 2.7~3.0 (3H, m), 3.0~3.1 (2H, m), 4.2~4.55 (1H, m), 5.6 (1H, dd, J = 10, 2Hz), 5.74~ 5.84 (1H, m)

Reference example 2

(1R*, 2R*, 6S*) - 2 - (N - tert - butoxycarbonyl - N - methylamino) - 8 - azabicyclo [4.3.0] non - 3 - ene

1)

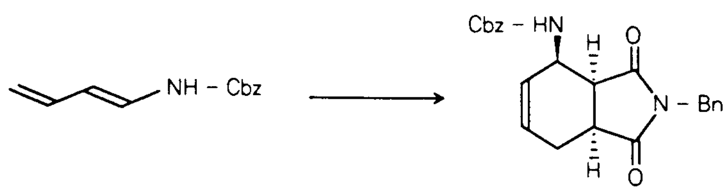

The procedure of Reference example 1 - 1) was repeated using (E) - 1 - (N - benzyloxycarbonylamino) - 1, 3 - butadiene [10.2g, 50mmol, prepared according to Organic Syntheses 6, 95 (1988)], N - benzylmaleinimide (9.91g, 53mmol) and 4 - tert - butylcatechol (100mg) to obtain (3R*, 8S*, 9S*) - 3 - (N - benzyloxycarbonylamino ) - 1 - benzyl - 3 , 6 , 8 , 9 - tetrahydrophthalimide (19.3g, yield : 98%).
TLC (Rf) : 0.5 (hexane - ethyl acetate 2 : 1)
IR (KBr) $cm^{-1}$ : 3400, 1700, 1510, 1400, 1340, 1240, 700
NMR ($CDCl_3$) δ : 2.21 (1H, dd, J = 15, 7Hz), 2.70 (1H, dd, J = 13, 7Hz), 3.1~3.35 (2H, m), 4.4~4.6 (1H, m), 4.6 (2H, s), 5.15 (2H s), 5.7~5.9 (2H, m), 6.5~6.65 (1H, br), 7.2~7.5 (10H, m)
2)

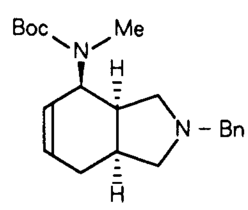

The procedure of Reference example 1 - 3) was repeated using the compound obtained by the above reaction (3.9g, 10mmol) and lithium aluminum hydride (2.4g, 63mmol) to obtain (1R*, 2R*, 6S*) - 8 - benzyl - 2 - (N - tert - butoxycarbonyl - N - methylamino) - 8 - azabicyclo [4.3.0] non - 3 - ene (668mg, yield : 19.5%).
TLC (Rf) : 0.25 (methylene chloride - methanol 9 : 1)
IR (KBr) $cm^{-1}$ : 3440, 2975, 2930, 2800, 1690, 1480, 1455, 1440, 1380, 1365, 1335, 1260, 1140, 700
NMR ($CDCl_3$) δ : 1.45 (9H, s), 1.95~3.10 (8H, m), 2.73 (3H, s), 3.69 (2H s), 4.7~5.0 (1H, m), 5.71 (1H, d, J = 10Hz), 5.8~ 6.0 (1H, m), 7.2~7.6 (5H, m)
3)

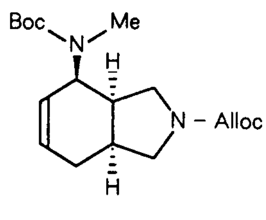

The procedure of Reference example 1 - 4) was repeated using the compound obtained by the above reaction (668mg, 1. 95mmol) and allyl chloroformate (0.42ml, 2mmol) to obtain (1R*, 2R*, 6S*) - 8 - allyloxycarbonyl - 2 - (N - tert - butoxycarbonyl - N - methylamino) - 8 - azabicyclo [4.3.0] non - 3 - ene (210mg, yield : 31.8%).
TLC (Rf) : 0.7 (methylene chloride - methanol 9 : 1)
IR (KBr) $cm^{-1}$ : 3400, 2980, 2940, 2890, 1700, 1440, 1410, 1370, 1340, 1145, 770
NMR ($CDCl_3$) δ : 1.46 (9H, s), 1.7~2.0 (1H, m), 2.0~2.3 (1H, m), 2.38~2.6 (1H, m), 2.71 (3H, s), 2.7~2.9 (1H, m), 3.2~3.55 (4H, m), 4.61 (2H, dd, J = 6, 1Hz), 4.9~5.6 (4H, m), 5.75~ 6.1 (2H, m)
4)

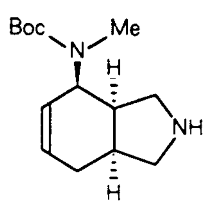

The procedure of Reference example 1 - 5) was repeated using the compound obtained by the above reaction (200mg, 0. 6mmol), acetic acid (86 μ l, 1.5mmol), bis (triphenylphosphine) palladium (II) chloride (8.5mg) and tributyltin hydride (323 μ l , 1 . 2mmol) to obtain (1R*, 2R*, 6S*) - 2 - (N - tert - butoxycarbonyl - N - methylamino) - 8 - azabicyclo [4.3.0] non - 3 - ene (99mg, yield : 65.5%).
TLC (Rf) : 0.07 (methylene chloride - methanol 9 : 1)
IR (KBr) $cm^{-1}$ : 3400, 2930, 2850, 1700, 1445, 1400, 1385, 1370, 1145
NMR ($CDCl_3$ - $CD_3OD$ 5:1) δ : 1.42 (9H, s), 1.8~2.0 (1H, m), 2.1~ 2.3 (1H, m), 2.4~3.1 (5H, m), 2.70 (3H, s), 4.9~5.1 (1H, m), 5.56 (1H, d, J = 10Hz), 5.8~5.95 (1H, m)

Reference example 3

(1R*, 2R*, 6S*) - 2 - amino - 8 - azabicyclo [4.3.0] non - 3 - ene dihydrochloride

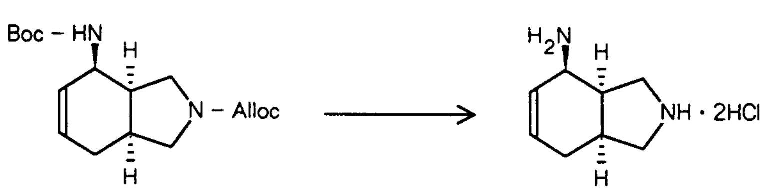

(1R*, 2R*, 6S*) - 8 - Allyloxycarbonyl - 2 - (N - tert - butoxycarbonylamino) - 8 - azabicyclo [4.3.0] non - 3 - ene (1.58g, 4.6mmol) obtained in Reference example 1 - 4) was refluxed with heating for 22 hours in 3N hydrochloric acid (20ml) with stirring. The solvent was distilled away under reduced pressure, ethanol was added, and the precipitate was taken by filtration which was the captioned compound (0.80g, yield : 82.4%).
TLC (Rf) : 0.05 (ethyl acetate - acetic acid - water 3 : 1 : 1)
IR (KBr) $cm^{-1}$ : 3400, 2950, 2050, 1610, 1520, 1390, 785, 680
NMR ($D_2O$) δ : 1.8~2.0 (1H, m), 2.35~2.55 (1H, m), 2.8~3.65 (6H, m), 4.35~4.5 (1H, m), 5.65~5.75 (1H, m), 6.0~6.15 (1H, m)

Reference example 4

1)
(1R*, 2R*, 6S*) - 2 - amino - 8 - azabicyclo [4.3.0] non - 3 - ene L - (+) - tartrate

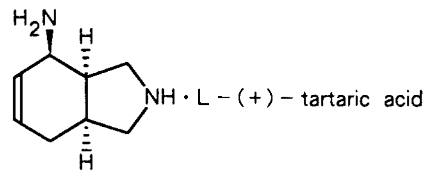

A 80% aqueous methanol solution (15ml) of (±) - (1R*, 2R*, 6S*) - 2- amino - 8 - azabicyclo [4.3.0] non - 3 - ene (2.1g, 15.2mmol) and L - (+) - tartaric acid (1.140g, 7.6mmol) was stirred at room temperature for 1 hour. The precipitate was taken by filtration and recrystallized from a 80 % aqueous methanol solution (70ml) to obtain the captioned compound (1.01g, yield : 46.0%) as scaly crystals.
$[\alpha]_D^{20}$ : + 40.3° (C = 1.0, $H_2O$)
IR (KBr) $cm^{-1}$ : 3340, 2930, 2240, 1595, 1550, 1400, 1320, 1105, 1075
NMR ($D_2O$) δ : 1.95~2.14 (1H, m), 2.5~2.7 (1H, m), 2.92~3.8 (6H, m), 4.54 (2H, s), 4.5~4.6 (1H, m), 5.83 (1H, br d, J = 10Hz), 6.15~6.28 (1H, m)

2)
(1R*, 2R*, 6S*) - 2 - amino - 8 - azabicyclo [4.3.0] non - 3 - ene D - (-) -tartrate

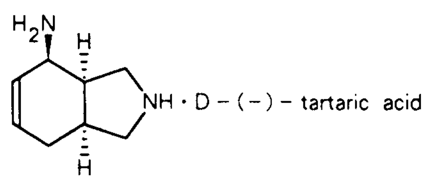

The mother liquors in the above reaction were combined, the solvents were distilled away under reduced pressure, and water (50ml) was added to the residue to give a solution, and the solution was subjected to IRA - 401 to obtain (±) - (1R*, 2R*, 6S*) - 2 - amino - 8 - azabicyclo [4.3.0] non - 3 - ene (1.40g, 10.1mmol). This was dissolved in a 80 % aqueous methanol solution (15ml), D - (-) - tartaric acid (1.140g, 7.6mmol) was added, and the mixture was thereafter treated in the same manner as in Reference example 4 - 1) to obtain the captioned compound (1.05g, yield : 48.0%) as scaly crystals.
$[\alpha]_D^{20}$ : - 42.4° (C = 1.0, $H_2O$)
IR (KBr) $cm^{-1}$ : 3420, 3260, 2950, 2930, 1640, 1550, 1400, 1380, 1120, 1060
NMR ($D_2O$) δ : 2.0~2.2 (1H, m), 2.5~2.7 (1H, m), 2.9~3.8 (6H, m), 4.48 (2H, s), 4.5~4.6 (1H, m), 5.84 (1H, br d, J = 10Hz), 6.15~6.3 (1H, m)

Industrial Applicability

The compounds of this invention are novel compounds not disclosed in literatures and useful as antitumor agents.

## Claims

**1.** Compounds represented by the following general formula or their pharmaceutically acceptable salts or

esters

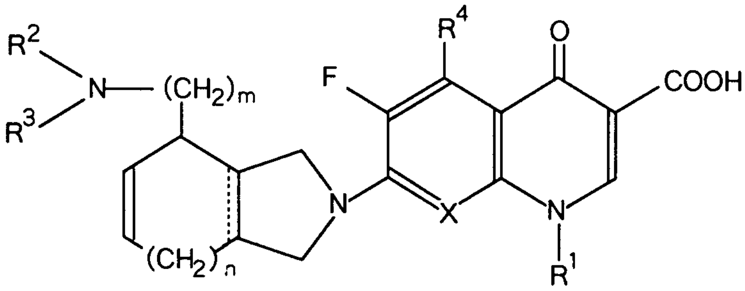

[I]

wherein $R^1$ represents a straight - chain, branched or cyclic lower alkyl group or a phenyl group optionally substituted by fluorine atom(s), $R^2$ and $R^3$ each represent a hydrogen atom or a straight - chain, branched or cyclic lower alkyl group, $R^4$ represents a hydrogen atom or - N ($R^5$) $R^6$ wherein $R^5$ and $R^6$ each represent a hydrogen atom or a straight - chain, branched or cyclic lower alkyl group, X represents a nitrogen atom or C - Y wherein Y represents a hydrogen atom, halogen atom, methyl group or methoxy group, m and n each represent an integer of 0 or 1, and ---- represents a single bond or double bond.

**2.** The compounds of claim 1 wherein $R^1$ is a cyclopropyl group.

**3.** The compound of claim 1 wherein $R^4$ is a hydrogen atom.

**4.** The compound of claim 1 wherein $R^4$ is - $NH_2$.

**5.** The compound of claim 1 wherein m is 0 and n is 1.

**6.** The compound of claim 1 represented by the general formula

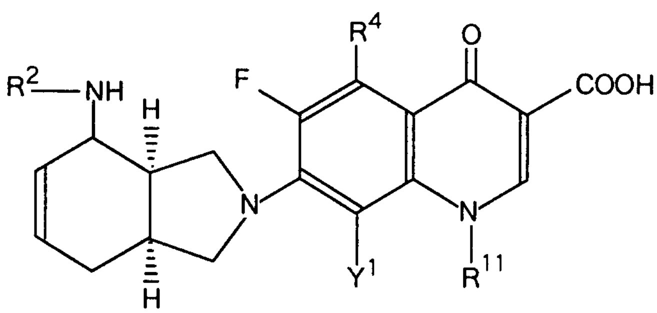

[I - a]

wherein $R^2$ represents a hydrogen atom or a straight - chain, branched or cyclic lower alkyl group, $R^4$ represents a hydrogen atom or - N ($R^5$) $R^6$ wherein $R^5$ and $R^6$ each represent a hydrogen atom or a straight - chain, branched or cyclic lower alkyl group, $R^{11}$ represents a straight - chain, branched or cyclic lower alkyl group, and $Y^1$ represents a hydrogen atom or halogen atom.

**7.** The compound of claim 6 represented by the general formula

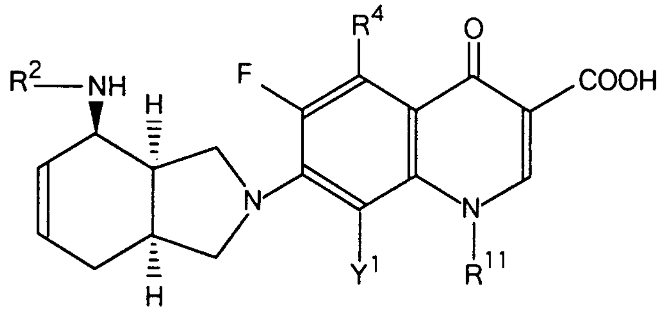

[I - b]

wherein $R^2$ is a hydrogen atom or a straight - chain, branched or cyclic lower alkyl group, $R^4$ represents a hydrogen atom or - N ($R^5$) $R^6$ wherein $R^5$ and $R^6$ each represent a hydrogen atom or a straight - chain, branched or cyclic lower alkyl group, $R^{11}$ represents a straight - chain, branched or cyclic lower alkyl group, and $Y^1$ represents a hydrogen atom or halogen atom.

**8.** The compounds of claim 1 which are

7 - [(1R*, 2R*, 6S*) -2 - amino - 8 - azabicyclo [4.3.0] non - 3 - en - 8 - yl] - 1 - cyclopropyl - 6, 8 - difluoro - 1, 4 - dihydro - 4 - oxo - 3 - quinolinecarboxylic acid,

1 -cyclopropyl - 6, 8 - difluoro - 1, 4 - dihydro - 7 - [(1R*, 2R*,6S*) - 2 - (N -methylamino) - 8 - azabicyclo [4.3.0] non - 3 - en - 8 - yl] - 4 - oxo - 3 - quinolinecarboxylic acid,

7 - [(1R*, 2R*, 6S*) - 2 - amino - 8 - azabicyclo [4.3.0] non - 3 - en - 8- yl] - 1 - cyclopropyl - 6 - fluoro - 1, 4 - dihydro - 4 - oxo - 3 - quinolinecarboxylic acid,

7 - [(1R*, 2R*, 6S*) - 2 - amino - 8 - azabicyclo [4.3.0] non - 3 - en - 8 - yl] - 6, 8 - difluoro - 1 - (2, 4 - difluorophenyl) - 1, 4 - dihydro- 4 - oxo - 3 - quinolinecarboxylic acid,

7 - [(1R*, 2R*, 6S*) - 2 - amino - 8 - azabicyclo [4.3.0] non - 3 - en - 8 - yl] - 1 - ethyl - 6 - fluoro- 1, 4- dihydro - 4 - oxo - 3 - quinolinecarboxylic acid,

7 - [(1R*, 2R*, 6S*) - 2 - amino - 8 - azabicyclo [4.3.0] non - 3 - en - 8 - yl] - 1 - cyclopropyl - 6 - fluoro - 1, 4 - dihydro - 8 - methoxy - 4 - oxo - 3 - quinolinecarboxylic acid and

5 - amino - 7 - [(1R*, 2R*, 6S*) - 2 - amino - 8 - azabicyclo [4.3.0] non - 3 - en - 8 - yl] - 1 - cyclopropyl - 6, 8 - difluoro - 1, 4 - dihydro - 4 - oxo - 3 - quinolinecarboxylic acid.

**9.** A process for preparation of a compound represented by the following general formula or a pharmaceutically acceptable salt or ester thereof

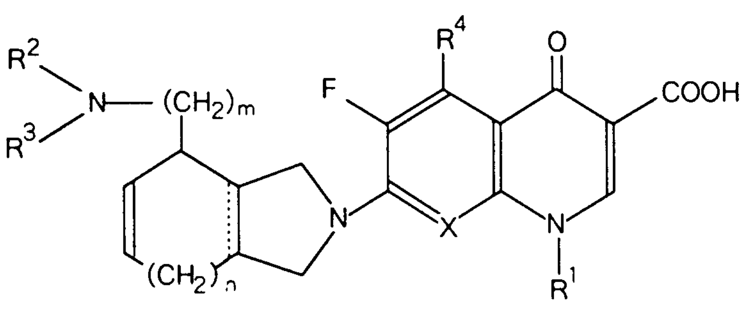

[I]

wherein $R^1$ represents a straight - chain, branched or cyclic lower alkyl group or a phenyl group optionally substituted by fluorine atom (s), $R^2$ and $R^3$ each represent a hydrogen atom or a straight - chain, branched or cyclic lower alkyl group, $R^4$ represents a hydrogen atom or - N ($R^5$) $R^6$ wherein $R^5$ and $R^6$ each represent a hydrogen atom or a straight - chain, branched or cyclic lower alkyl group, X represents a nitrogen atom or C - Y wherein Y represents a hydrogen atom, halogen atom, methyl group or methoxy group, m and n each represent an integer of 0 or 1, and ═ represents a single bond or double bond, which comprises reacting a compound represented by the following general formula or a salt thereof

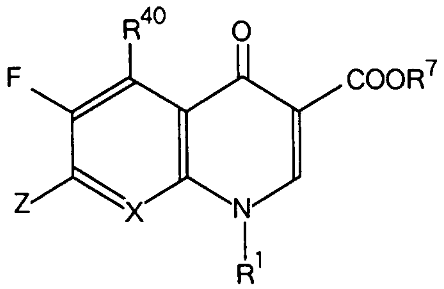

[IV]

wherein $R^7$ represents a hydrogen atom or a protective group for a carboxyl group, $R^{40}$ represents a hydrogen atom or - N ($R^{50}$) $R^{60}$ wherein $R^{50}$ and $R^{60}$ each represent a hydrogen atom, a protective group for an amino group, or a straight - chain, branched or cyclic lower alkyl group, Z represents an eliminable group, and $R^1$ and X have the same meanings as defined above, with a compound represented by the following general formula or a salt thereof

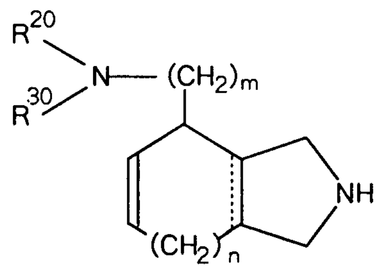

[III]

wherein $R^{20}$ and $R^{30}$ each represent a hydrogen atom, a protective group for an amino group, or a straight - chain, branched or cyclic lower alkyl group, and m, n and ═══ have the same meanings as defined above,
to give a compound represented by the general formula

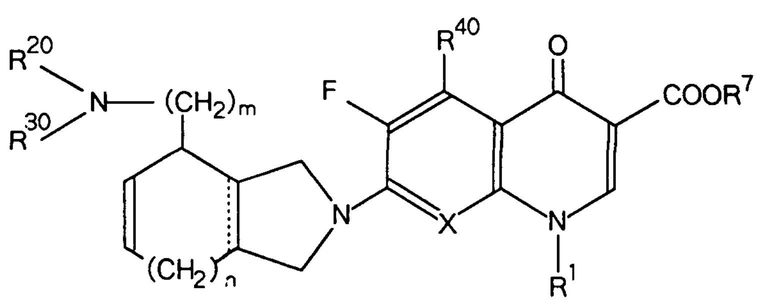

[II]

wherein $R^1$, $R^7$, $R^{20}$, $R^{30}$, $R^{40}$, X, m, n and ═══ have the same meanings as defined above,
and then, if necessary, removing the protective groups of the compound.

**10.** An antitumor agent containing as an effective ingredient a compound represented by the following general formula or a pharmaceutically acceptable salt or ester thereof

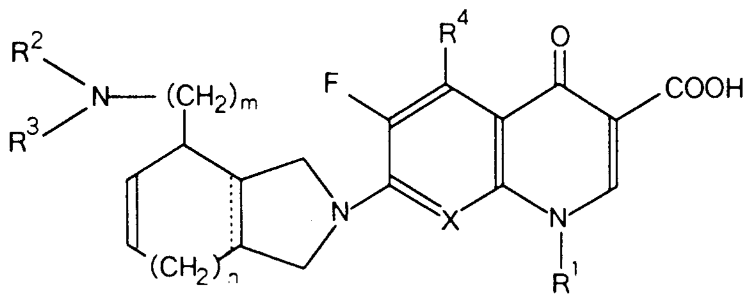

[ I ]

wherein $R^1$ represents a straight - chain, branched or cyclic lower alkyl group or a phenyl group optionally substituted by fluorine atom(s), $R^2$ and $R^3$ each represent a hydrogen atom or a straight - chain, branched or cyclic lower alkyl group, $R^4$ represents a hydrogen atom or - N ($R^5$) $R^6$ wherein $R^5$ and $R^6$ each represent a hydrogen atom or a straight - chain, branched or cyclic lower alkyl group, X represents a nitrogen atom or C - Y wherein Y represents a hydrogen atom, halogen atom, methyl group or methoxy group, m and n each represent an integer of 0 or 1, and ⩶ represents a single bond or double bond.

# INTERNATIONAL SEARCH REPORT

International Application No PCT/JP91/01640

**I. CLASSIFICATION OF SUBJECT MATTER** (if several classification symbols apply, indicate all) [6]

According to International Patent Classification (IPC) or to both National Classification and IPC

Int. $Cl^5$ C07D401/04, 471/04

**II. FIELDS SEARCHED**

Minimum Documentation Searched [7]

| Classification System | Classification Symbols |
|---|---|
| IPC | C07D401/04, 471/04 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 02-56479 (Shionogi & Co., Ltd.), February 26, 1990 (26. 02. 90), & EP, A1, 343524 | 1-10 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 31, 1992 (31. 01. 92) | March 3, 1992 (03. 03. 92) |
| **International Searching Authority** | **Signature of Authorized Officer** |
| Japanese Patent Office | |